(19) **Europäisches Patentamt / European Patent Office / Office européen des brevets**

(11) **EP 3 043 902 B1**

(12) **FASCICULE DE BREVET EUROPEEN**

(45) Date de publication et mention de la délivrance du brevet:
**27.07.2022 Bulletin 2022/30**

(21) Numéro de dépôt: **14761826.8**

(22) Date de dépôt: **05.09.2014**

(51) Classification Internationale des Brevets (IPC):
*B01J 20/18* (2006.01)     *B01J 20/28* (2006.01)
*B01J 20/30* (2006.01)     *B01J 20/12* (2006.01)
*B01J 20/10* (2006.01)     *B01D 53/02* (2006.01)
*B01D 53/08* (2006.01)     *C07C 7/13* (2006.01)
*C07C 37/82* (2006.01)     *C07C 201/16* (2006.01)
*C07C 209/86* (2006.01)

(52) Classification Coopérative des Brevets (CPC):
(C-Sets disponibles)
**B01J 20/18; B01D 53/02; B01D 53/08; B01J 20/12;
B01J 20/186; B01J 20/28004; B01J 20/28059;
B01J 20/28061; B01J 20/28071; B01J 20/3028;
B01J 20/3071; B01J 20/3085; C07C 7/13;
C07C 37/82; C07C 201/16;** (Cont.)

(86) Numéro de dépôt international:
**PCT/EP2014/068993**

(87) Numéro de publication internationale:
**WO 2015/032923 (12.03.2015 Gazette 2015/10)**

(54) **ADSORBANTS ZÉOLITHIQUES DE HAUTE SURFACE EXTERNE COMPRENANT DU BARYUM ET/OU DUE POTASSIUM ET LEURS UTILISATIONS**

ZEOLITHISCHE ADSORPTIONSMITTEL MIT GROSSEN EXTERNEN OBERFLÄCHE UMFASSEND BARIUM UND/ODER KALIUM UND VERWENDUNGEN DAVON

ZEOLITIC ADSORBENTS WITH LARGE EXTERNAL SURFACE AREA COMPRISING BARYUM AND/OR POTASSIUM AND USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.09.2013 FR 1358662**
**10.09.2013 FR 1358715**

(43) Date de publication de la demande:
**20.07.2016 Bulletin 2016/29**

(73) Titulaires:
• **ARKEMA FRANCE**
**92700 Colombes (FR)**
• **IFP Energies nouvelles**
**92500 Rueil-Malmaison (FR)**

(72) Inventeurs:
• **LAROCHE, Catherine**
**F-69390 Vernaison (FR)**
• **LEFLAIVE, Philibert**
**F-69780 Mions (FR)**
• **BOUVIER, Ludivine**
**F-64300 Orthez (FR)**
• **NICOLAS, Serge**
**F-64140 Lons (FR)**
• **LUTZ, Cécile**
**F-64290 Gan (FR)**
• **LABÈDE, Marie-Laurence**
**F-64230 Lescar (FR)**

(74) Mandataire: **Bandpay & Greuter**
**30, rue Notre-Dame des Victoires**
**75002 Paris (FR)**

(56) Documents cités:
**FR-A1- 2 789 914      FR-A1- 2 903 978**
**US-A1- 2012 247 334**

- **Polaert Isabelle ET AL: "Adsorbents regeneration under microwave irradiation for dehydration and volatile organic compounds gas treatment", Chemical Engeneering Journal, vol. 162, no. 3, 6 July 2010 (2010-07-06), pages 941-948, XP055841830, AMSTERDAM, NL ISSN: 1385-8947, DOI: 10.1016/j.cej.2010.06.047**

(52) Classification Coopérative des Brevets (CPC):
(Cont.)
**C07C 209/86;** B01D 2253/108; B01D 2257/556

C-Sets
**C07C 7/13, C07C 15/02;**
**C07C 7/13, C07C 15/08;**
**C07C 37/82, C07C 39/07;**
**C07C 201/16, C07C 205/06;**
**C07C 209/86, C07C 211/50**

**Description**

L'invention concerne des adsorbants sous forme d'agglomérés comprenant de la zéolithe de type faujasite, adsorbants présentant une importante surface externe caractérisée par adsorption d'azote, typiquement supérieure à 20 $m^2.g^{-1}$, et de préférence comprise entre 40 $m^2.g^{-1}$ et 200 $m^2.g^{-1}$.

La présente invention concerne également leurs utilisations pour la séparation de mélanges gazeux ou liquides d'isomères, plus particulièrement des xylènes et notamment pour la production de paraxylène très pur à partir d'une charge d'hydrocarbures aromatiques contenant des isomères à 8 atomes de carbone.

L'utilisation d'adsorbants zéolithiques constitués de zéolithes Faujasite (FAU) de type X ou Y comprenant, outre des cations sodium, des ions baryum, potassium ou strontium, seuls ou en mélanges, pour adsorber sélectivement le *para*-xylène dans un mélange d'hydrocarbures aromatiques, est bien connue de l'art antérieur.

Les brevets US3558730, US3558732, US3626020 et US3663638 montrent que des adsorbants zéolithiques comprenant des aluminosilicates à base de sodium et de baryum (US3960774) ou à base de sodium, de baryum et de potassium, sont efficaces pour la séparation du *para*-xylène présent dans des coupes aromatiques en C8 (coupes comprenant des hydrocarbures aromatiques à 8 atomes de carbone).

Les adsorbants décrits dans le brevet US3878127 sont utilisés comme agents d'adsorption dans les procédés en phase liquide, de préférence de type contre-courant simulé, similaires à ceux décrits dans le brevet US2985589 et qui s'appliquent, entre autres, aux coupes aromatiques en C8.

Dans les brevets listés ci-dessus, les adsorbants zéolithiques se présentent sous forme de cristaux à l'état de poudre ou sous forme d'agglomérés constitués majoritairement de poudre de zéolithe et jusqu'à 20% en poids de liant inerte.

La synthèse des zéolithes FAU s'effectue habituellement par nucléation et cristallisation de gels de silico-aluminates. Cette synthèse conduit à des cristaux (généralement sous forme de poudre) dont l'emploi à l'échelle industrielle est particulièrement malaisé (pertes de charges importantes lors des manipulations). On préfère alors les formes agglomérées de ces cristaux, sous forme de grains, de filés et autres agglomérés, ces dites formes pouvant être obtenues par extrusion, pastillage, atomisation et autres techniques d'agglomération connues de l'homme du métier. Ces agglomérés ne présentent pas les inconvénients inhérents aux matières pulvérulentes.

D'autre part, les cristaux de zéolithes sont le plus souvent préparés à partir de solutions aqueuses sodiques (par exemple solution aqueuse d'hydroxyde de sodium), et, si on le souhaite, les cations sodium peuvent être remplacés (échangés) en totalité ou en partie par d'autres cations, par exemple baryum ou baryum et potassium. Ces échanges cationiques peuvent être effectués avant et/ou après agglomération de la zéolithe pulvérulente avec le liant d'agglomération, selon des techniques classiques connues de l'homme du métier.

Les agglomérés, qu'ils soient sous forme de plaquettes, de billes, d'extrudés, et autres, sont en général constitués de cristaux de zéolithe(s), qui constituent l'élément actif (au sens de l'adsorption) et d'un liant d'agglomération. Ce liant d'agglomération est destiné à assurer la cohésion des cristaux entre eux dans la structure agglomérée, mais aussi doit permettre d'assurer une résistance mécanique suffisante auxdits agglomérés afin d'éviter, ou tout au moins de minimiser le plus possible, les risques de fractures, brisures ou cassures qui pourraient survenir lors de leurs utilisations industrielles pendant lesquelles les agglomérés sont soumis à de nombreuses contraintes, telles que vibrations, variations fortes et/ou fréquentes de pressions, mouvements et autres.

La préparation de ces agglomérés s'opère par exemple par empâtage de cristaux de zéolithe à l'état de poudre avec une pâte argileuse, dans des proportions de l'ordre de 80 à 90 % en poids de poudre de zéolithe pour 20% à 10% en poids de liant, puis mise en forme en billes, plaquettes ou extrudés, et traitement thermique à haute température pour cuisson de l'argile et réactivation de la zéolithe, le ou les échange(s) cationique(s), comme par exemple l'échange au baryum pouvant être effectué avant et/ou après l'agglomération de la zéolithe pulvérulente avec le liant.

On obtient des corps zéolithiques dont la granulométrie est de quelques millimètres, voire de l'ordre du millimètre, et qui, si le choix du liant d'agglomération et la granulation sont faits dans les règles de l'art, présentent un ensemble de propriétés satisfaisantes, en particulier de porosité, de résistance mécanique, de résistance à l'abrasion. Cependant, les propriétés d'adsorption de ces agglomérés sont évidemment réduites par rapport à la poudre active de départ en raison de la présence de liant d'agglomération inerte vis-à-vis de l'adsorption.

Divers moyens ont déjà été proposés pour pallier cet inconvénient du liant d'agglomération d'être inerte quant aux performances d'adsorption, parmi lesquels, la transformation de la totalité ou d'au moins une partie du liant d'agglomération en zéolithe active du point de vue de l'adsorption. Cette opération est maintenant bien connue de l'homme du métier, par exemple sous la dénomination de « zéolithisation ». Pour effectuer facilement cette opération, on utilise des liants zéolithisables, le plus souvent appartenant à la famille de la kaolinite, et de préférence préalablement calcinés à des températures généralement comprises entre 500°C et 700°C.

La demande de brevet FR2789914 décrit un procédé de fabrication d'agglomérés de zéolithe X, de rapport atomique Si/Al compris entre 1,15 et 1,5, échangés au baryum et éventuellement au potassium, en agglomérant des cristaux de zéolithe X avec un liant, une source de silice et de la carboxyméthylcellulose, puis en zéolithisant le liant par immersion de l'aggloméré dans une liqueur alcaline. Après échange des cations de la zéolithe par des ions baryum (et éventuellement potassium) et activation, les agglomérés ainsi obtenus présentent, du point de vue de l'adsorption du *para*-xylène contenu dans les coupes aromatiques en C8, des propriétés améliorées par rapport à des adsorbants préparés à partir de la même quantité de zéolithe X et de liant, mais dont le liant n'est pas zéolithisé.

Outre une capacité d'adsorption élevée et de bonnes propriétés de sélectivité vis-à-vis de l'espèce à séparer du mélange réactionnel, l'adsorbant doit présenter de bonnes propriétés de transfert de matière afin de garantir un nombre de plateaux théoriques suffisants pour réaliser une séparation efficace des espèces en mélange, comme l'indique Ruthven dans l'ouvrage intitulé « Principles of Adsorption and Adsorption Processes » (« Principes de l'Adsorption et des Procédés d'Adsorption »), John Wiley & Sons, (1984), pages 326 et 407. Ruthven indique *(ibid.,* page 243), que, dans le cas d'un adsorbant aggloméré, le transfert de matière global dépend de l'addition de la résistance diffusionnelle intra-cristalline et de la résistance diffusionnelle entre les cristaux.

La résistance diffusionnelle intra-cristalline est proportionnelle au carré des diamètres des cristaux et inversement proportionnelle à la diffusivité intracristalline des molécules à séparer.

La résistance diffusionnelle entre les cristaux (également appelée « résistance macroporeuse ») est quant à elle proportionnelle au carré des diamètres des agglomérés, inversement proportionnelle à la porosité contenue dans les macropores et mésopores (c'est-à-dire les pores dont l'ouverture est supérieure à 2 nm) au sein de l'aggloméré, et inversement proportionnelle à la diffusivité des molécules à séparer dans cette porosité.

La taille des agglomérés est un paramètre important lors de l'utilisation de l'adsorbant dans l'application industrielle, car elle détermine la perte de charge au sein de l'unité industrielle et l'uniformité du remplissage. La distribution granulométrique des agglomérés doit donc être étroite, et centrée sur des diamètres moyens en nombre compris typiquement entre 0,40 mm et 0,65 mm afin d'éviter des pertes de charges excessives. La porosité contenue dans les macropores et mésopores ne participe pas à la capacité d'adsorption. Par conséquent, l'homme du métier ne cherchera pas à l'augmenter dans le but de réduire la résistance diffusionnelle macroporeuse, sachant que cela se ferait au détriment de la capacité d'adsorption volumique.

Pour estimer l'amélioration de la cinétique de transfert, il est possible d'utiliser la théorie des plateaux décrite par Ruthven dans « *Principles of Adsorption and Adsorption Processes* », *ibid.,* pages 248-250. Cette approche est basée sur la représentation d'une colonne par un nombre fini de réacteurs hypothétiques idéalement agités (étages théoriques). La hauteur équivalente de plateaux théoriques est une mesure directe de la dispersion axiale et de la résistance au transfert de matière du système.

Pour une structure zéolithique donnée, une taille d'aggloméré donnée et une température de fonctionnement donnée, les diffusivités sont fixées, et un des moyens d'améliorer le transfert de matière consiste à réduire le diamètre des cristaux. Un gain sur le transfert de matière global sera ainsi obtenu en réduisant la taille des cristaux.

L'homme du métier va donc chercher à réduire le plus possible le diamètre des cristaux de zéolithes afin d'améliorer le transfert de matière.

Le brevet CN1267185C, revendique ainsi des adsorbants contenant 90% à 95% de zéolithe BaX ou BaKX pour la séparation du para-xylène, dans lesquels les cristaux de zéolithe X sont de taille comprise entre 0,1 $\mu$m et 0,4 $\mu$m et ceci afin d'améliorer les performances en transfert de matière. De même, le brevet US20090326308 décrit un procédé de séparation des isomères du xylène dont les performances ont été améliorées par l'utilisation d'adsorbants à base de cristaux de zéolithe X de taille inférieure à 0,5 $\mu$m.

La demanderesse a néanmoins observé que la synthèse, la filtration, la manipulation et l'agglomération de cristaux de zéolithe dont la taille est inférieure à 0,5 $\mu$m mettent en oeuvre des procédés lourds, peu économiques et donc difficilement industrialisables.

De plus, de tels agglomérés comportant des cristaux de taille inférieure à 0,5 $\mu$m, s'avèrent également plus fragiles, et il devient alors nécessaire d'augmenter le taux de liant d'agglomération afin de renforcer la cohésion des cristaux entre eux au sein de l'aggloméré. Toutefois, l'augmentation du taux de liant d'agglomération conduit à une densification des agglomérés, à l'origine d'une augmentation de la résistance diffusionnelle macroporeuse. Ainsi, malgré une résistance diffusionnelle intra-cristalline réduite du fait de la diminution de la taille des cristaux, l'augmentation de la résistance diffusionnelle macroporeuse en raison de la densification de l'aggloméré, ne permet pas une amélioration du transfert global.

Il reste par conséquent un besoin pour des matériaux adsorbants zéolithiques préparés à partir de zéolithe de type FAU facilement manipulable au niveau industriel, c'est-à-dire dont les éléments cristallisés constitutifs (ou plus simplement les « cristaux ») sont avantageusement de taille supérieure à 0,5 $\mu$m, mais présentant un transfert de matière global amélioré par rapport à un adsorbant préparé à partir de cristaux de zéolithe de type FAU conventionnels de taille identique (c'est-à-dire supérieure à 0,5 $\mu$m), tout en conservant une capacité d'adsorption élevée.

Ces adsorbants améliorés seraient ainsi particulièrement adaptés à la séparation des isomères des xylènes en phase gaz ou en phase liquide.

La présente invention a ainsi pour premier objet de proposer des adsorbants zéolithiques sous forme d'agglomérés aux propriétés optimisées pour la séparation de mélanges gazeux ou liquides d'isomères et plus particulièrement pour la séparation des xylènes, en phase gaz ou en phase liquide, notamment du *para*-xylène des coupes aromatiques en C8. Les adsorbants zéolithiques de l'invention présentent notamment des propriétés maximales de sélectivité vis-à-vis du *para*-xylène et de transfert de matière, tout en présentant une résistance mécanique et une capacité d'adsorption élevées et sont particulièrement adaptés pour une utilisation dans un procédé de séparation du *para*-xylène en phase liquide, de préférence de type contre-courant simulé.

Plus précisément, la présente invention est selon la revendication 1.

Selon encore un autre mode de réalisation de l'invention, l'adsorbant zéolithique présente une teneur en oxyde de baryum (BaO) supérieure à 10%, de préférence supérieure à 15%, de manière très préférée supérieure à 20%, de manière encore plus préférée supérieure à 23 %, voire supérieure à 33% en poids par rapport à la masse totale de l'adsorbant. Avantageusement, la teneur en baryum est comprise entre 23% et 42%, et typiquement entre 30% et 40%, bornes incluses, en poids par rapport au poids total de l'adsorbant.

Selon un autre mode réalisation de l'invention, l'adsorbant zéolithique peut présenter une teneur en oxyde de potassium $K_2O$ inférieure à 25%, de préférence comprise entre 0 et 20%, de manière encore plus préférée comprise entre 0% et 15% et de manière très préférée de 0% à 10% bornes incluses en poids par rapport à la masse totale de l'adsorbant.

Selon un autre mode réalisation de l'invention la teneur totale en oxydes d'ions alcalins ou alcalino-terreux autres que l'oxyde de baryum BaO et l'oxyde de potassium $K_2O$ est comprise entre 0 et 5%, bornes incluses, par rapport à la masse totale de l'adsorbant.

De préférence l'adsorbant zéolithique selon la présente invention est un adsorbant à base de zéolithe(s) FAU, généralement référencée(s) sous le nom de zéolithe de type X. Par « zéolithe X », on entend une zéolithe dont le rapport atomique Si/Al est compris entre 1,00 et 1,50 bornes incluses, de préférence entre 1,05 et 1,50, de préférence entre 1,05 et 1,40, bornes incluses et de manière encore plus préférée entre 1,10 et 1,40 bornes incluses.

Parmi les zéolithes X, il est maintenant communément admis de reconnaître deux sous-groupes dénommés zéolithes LSX et zéolithes MSX. Les zéolithes LSX présentent un rapport atomique Si/Al égal à environ 1 et les zéolithes MSX présentent un rapport atomique Si/Al compris entre environ 1,05 et environ 1,15, bornes incluses.

Selon un mode de réalisation préféré de la présente invention, la zéolithe X présente un rapport atomique Si/Al compris entre 1,10 et 1,50, bornes incluses. Selon un autre mode de réalisation préféré, la zéolithe X est une zéolithe de type LSX de rapport atomique Si/Al est égal à environ 1. Il peut également être envisageable que l'adsorbant contienne des mélanges de deux ou plusieurs types de zéolithes X tels qu'ils viennent d'être définis.

Selon un mode de réalisation préféré, la au moins une zéolithe FAU comprise dans l'adsorbant zéolithique de l'invention présente un rapport atomique Si/Al compris entre 1,00 et 1,50 bornes incluses, de préférence entre 1,05 et 1,40 et de manière encore plus préférée compris entre 1,10 et 1,40. De préférence, la au moins une zéolithe FAU est une zéolithe X.

Selon un autre mode de réalisation préféré, aucune structure zéolithique autre que la structure FAU, de préférence aucune structure zéolithique autre que la structure faujasite X, n'est détectée par diffraction des rayons X (connue de l'homme du métier sous l'acronyme DRX) dans l'adsorbant zéolithique de la présente invention.

Selon encore un autre mode de réalisation préféré, la fraction massique de zéolithe FAU, la zéolithe FAU étant de préférence une zéolithe X, est supérieure ou égale à 80% par rapport au poids total d'adsorbant de la présente invention, le complément à 100% étant de préférence constitué de phase non zéolithique.

Les adsorbants zéolithiques selon la présente invention peuvent contenir une phase non zéolithique (PNZ), c'est-à-dire une phase non cristalline qui est essentiellement inerte vis-à-vis de l'adsorption. Le taux de cristallinité (fraction massique de zéolithe) de l'adsorbant selon l'invention peut être mesuré par analyse par diffraction de rayons X, connue de l'homme du métier sous l'acronyme DRX.

Selon un mode de réalisation préféré, l'adsorbant zéolithique selon l'invention présente une perte au feu mesurée à 900°C selon la norme NF EN 196-2 inférieure ou égale à 7,7%, de préférence comprise entre 0 et 7,7%, de manière préférée entre 3,0% et 7,7%, de manière encore préférée entre 3,5% et 6,5% et avantageusement entre 4,5% et 6%, bornes incluses.

L'adsorbant zéolithique selon la présente invention présente avantageusement une résistance mécanique et une capacité d'adsorption élevées. La résistance mécanique est mesurée par la méthode Shell série SMS1471-74 adaptée pour des agglomérés de taille inférieure à 1,6 mm et est généralement supérieure ou égale à 2 MPa, typiquement supérieure ou égale à 2,1 MPa.

L'adsorbant zéolithique de l'invention comprend de préférence à la fois des macropores, des mésopores et des micropores. Par « macropores », on entend des pores dont l'ouverture est supérieure à 50 nm, de préférence comprise entre 50 nm et 400 nm. Par « mésopores », on entend des pores dont l'ouverture est comprise entre 2 nm et 50 nm, bornes non incluses. Par « micropores », on entend des pores dont l'ouverture est inférieure à 2 nm.

Les mésopores de l'adsorbant zéolithique selon l'invention sont facilement identifiables par observation au moyen d'un microscope électronique à transmission (MET ou « TEM » en langue anglaise), comme décrit par exemple dans US7785563.

Selon encore un autre mode de réalisation préféré, l'adsorbant zéolithique de la présente invention se caractérise par un volume total des macropores et des mésopores, mesuré par intrusion de mercure, avantageusement compris entre 0,15 cm$^3$.g$^{-1}$ et 0,50 cm$^3$.g$^{-1}$, de préférence compris entre 0,20 cm$^3$.g$^{-1}$ et 0,40 cm$^3$.g$^{-1}$ et de manière très préférée compris entre 0,20 cm$^3$.g$^{-1}$ et 0,35 cm$^3$.g$^{-1}$.

La fraction en volume des macropores est de préférence comprise entre 0,2 et 1 du volume total des macropores et des mésopores, de manière très préférée comprise entre 0,4 et 0,8, et de manière encore plus préférée entre 0,45 et 0,65, bornes incluses.

On préfère également dans le cadre de la présente invention un adsorbant zéolithique dont le volume microporeux évalué par la méthode de t-plot à partir de l'isotherme d'adsorption d'azote ($N_2$) à une température de 77 K, est compris entre 0,180 cm$^3$.g$^{-1}$ et 0,290 cm$^3$.g$^{-1}$. Ladite mesure de volume microporeux est calculée après dégazage sous vide (P < 6,7.10$^{-4}$ Pa), à une température comprise entre 300°C et 450°C pendant une durée allant de 9 heures à 16 heures, de préférence à 400°C pendant 10 heures.

L'adsorbant zéolithique de l'invention est sous la forme d'un aggloméré, c'est-à-dire qu'il est constitué d'éléments cristallins (ou cristaux) d'au moins une zéolithe FAU telle que définie précédemment, lesdits éléments cristallins présentant un diamètre moyen en nombre compris entre 0,1 $\mu$m et 20 $\mu$m, de préférence entre 0,5 $\mu$m et 20 $\mu$m, de préférence encore entre 0,5 $\mu$m et 10 $\mu$m, et plus préférentiellement encore entre 0,5 $\mu$m et 5 $\mu$m, bornes incluses.

Selon encore un autre mode de réalisation préféré, l'adsorbant zéolithique selon l'invention comprend des éléments cristallins de zéolithe FAU de type X et ledit adsorbant zéolithique présente un rapport atomique Si/Al compris entre 1,00 et 2,00 de préférence entre 1,00 et 1,80 bornes incluses, de préférence encore entre 1,15 et 1,80, bornes incluses et de manière encore plus préférée entre 1,15 et 1,60 bornes incluses.

Comme indiqué précédemment, l'adsorbant zéolithique selon l'invention comprend en outre de préférence au moins une phase non zéolithique (PNZ) qui est utilisée dans le mode de préparation comme liant d'agglomération permettant la cohésion des éléments cristallins entre eux, avant d'être optionnellement tout ou partiellement zéolithisé, c'est-à-dire transformé en zéolithe active pour l'adsorption des molécules considérées, c'est-à-dire converti en zéolithe de type FAU.

Ceci signifie que l'adsorbant zéolithique selon l'invention comprend en outre et de préférence au moins une Phase Non Zéolithique (PNZ) qui comprend entre autres un liant d'agglomération utilisé dans le mode de préparation pour assurer la cohésion des cristaux entre eux, d'où le terme « aggloméré » ou « aggloméré zéolithique » employé parfois en lieu et place du terme « adsorbant zéolithique » de l'invention, tel que décrit précédemment.

Il a en effet été constaté par la demanderesse que des adsorbants zéolithiques FAU préparés à partir d'éléments cristallins de haute surface externe présentent un transfert de matière global amélioré par rapport à des adsorbants zéolithiques de type FAU préparés à partir de cristaux conventionnels, y compris lorsque les éléments cristallins sont de taille supérieure aux cristaux conventionnels.

La présente invention permet donc la mise à disposition d'adsorbants zéolithiques aux propriétés améliorées par rapport à l'art antérieur tout en facilitant la filtration, la manipulation et l'agglomération des poudres zéolithiques utilisées lors du procédé de fabrication.

Un procédé de préparation desdits adsorbants zéolithiques tels qu'ils viennent d'être définis, au moins les étapes de : comprend

a) agglomération d'éléments cristallins d'au moins une zéolithe de type FAU, présentant une surface externe supérieure à 40 m$^2$.g$^{-1}$, de préférence comprise entre 40 m$^2$.g$^{-1}$ et 400 m$^2$.g$^{-1}$, de préférence encore comprise entre 60 m$^2$.g$^{-1}$ et 200 m$^2$.g$^{-1}$, bornes incluses, de diamètre moyen en nombre compris entre 0,1 $\mu$m et 20 $\mu$m, de préférence entre 0,5 $\mu$m et 20 $\mu$m, de préférence encore entre 0,5 $\mu$m et 10 $\mu$m, et plus préférentiellement encore entre 0,5 $\mu$m et 5 $\mu$m bornes incluses, avec un liant comprenant au moins 80% d'argile ou d'un mélange d'argiles, éventuellement zéolithisable(s), et avec jusqu'à 5% d'additifs ainsi qu'avec la quantité d'eau qui permet la mise en forme du matériau aggloméré ; séchage des agglomérats à une température comprise entre 50°C et 150°C ; calcination des agglomérats séchés sous balayage gazeux oxydant et/ou inerte, avec notamment des gaz tels que l'oxygène, l'azote, l'air, un air sec et/ou décarbonaté, un air appauvri en oxygène, éventuellement sec et/ou décarbonaté, à une température supérieure à 150°C, typiquement comprise entre 180°C et 800°C, préférentiellement entre 200°C et 650°C, pendant quelques heures, par exemple de 2 heures à 6 heures ;

b) éventuellement zéolithisation de tout ou partie du liant par mise en contact des agglomérats obtenus à l'étape a) avec une solution basique alcaline ;

c) échange(s) cationique(s) des agglomérats de l'étape a) et/ou de l'étape b) par mise en contact avec une solution d'ions baryum et/ou d'ions potassium ;

d) échange cationique éventuel supplémentaire des agglomérats de l'étape c) par mise en contact avec une solution d'ions potassium ;

e) lavage et séchage des agglomérats obtenus aux étapes c) ou d), à une température comprise entre 50°C et 150°C ; et

f) obtention de l'adsorbant zéolithique selon l'invention par activation des agglomérats obtenus à l'étape e) sous balayage gazeux oxydant et/ou inerte, avec notamment des gaz tels que l'oxygène, l'azote, l'air, un air sec et/ou décarbonaté, un air appauvri en oxygène, éventuellement sec et/ou décarbonaté, à une température comprise entre 100°C et 400°C, de préférence entre 200°C et 300°C pendant une durée déterminée en fonction de la teneur en eau et de la perte au feu souhaitées, typiquement de 1 heure à 6 heures.

Selon un mode de réalisation préféré, ladite au moins une zéolithe FAU présente un rapport atomique Si/Al compris de préférence entre 1,00 et 1,50, de préférence entre 1,05 et 1,40 et de manière encore plus préférée compris entre 1,10 et 1,40, bornes incluses. Comme indiqué précédemment, la au moins une zéolithe FAU est de préférence une zéolithe X.

Comme indiqué précédemment, la surface externe des éléments cristallins mis en œuvre dans l'étape a) du procédé décrit ci-dessus est calculée par la méthode du t-plot à partir de l'isotherme d'adsorption d'azote à une température de 77 K, après dégazage sous vide (P < $6{,}7.10^{-4}$ Pa), à une température comprise entre 300°C et 450°C pendant une durée allant de 9 heures à 16 heures, de préférence à 400°C pendant 10 heures.

Les éléments cristallins de la zéolithe FAU présentant une importante surface externe mise en oeuvre à l'étape a) peuvent être obtenus selon diverses méthodes connues de l'homme du métier et par exemple selon la synthèse décrite par Inayat et coll. dans Angew. Chem. Int. Ed., (2012), 51, 1962-1965.

Il est également possible de préparer lesdits éléments cristallins par synthèse par ensemencement et/ou par ajustement des conditions opératoires de synthèse tels que le rapport $SiO_2/Al_2O_3$, la teneur en sodium et l'alcalinité du mélange de synthèse ou encore selon des procédés de post-traitement de cristaux de zéolithe FAU conventionnels.

Les procédés de post-traitements consistent généralement à éliminer des atomes du réseau zéolithique déjà formé, soit par un ou plusieurs traitements acides qui désaluminent le solide, traitement(s) suivi(s) par un ou plusieurs lavage(s) à la soude afin d'éliminer les résidus aluminiques formés, comme décrit par exemple par D. Verboekend, G. Vilé et J. Pérez-Ramírez, dans Adv. Funct. Mater., 22, (2012), p 916-928), soit encore par des traitements qui associent l'action d'un acide et celle d'un agent structurant qui améliorent l'efficacité du traitement acide, comme décrit par exemple dans la demande WO2013/106816.

Les procédés de synthèse directe des ces zéolithes (c'est-à-dire des procédés de synthèse autre que le post-traitement) font généralement intervenir un ou plusieurs agents structurants ou gabarits sacrificiels.

Les gabarits sacrificiels pouvant être utilisés peuvent être de tout type connu de l'homme du métier et notamment ceux décrits dans la demande WO2007/043731. Selon un mode de réalisation préféré, le gabarit sacrificiel est avantageusement choisi parmi les organosilanes et plus préférentiellement parmi le chlorure de [3-(triméthoxysilyl)propyl]-octadecyldiméthylammonium, le chlorure de [3-(triméthoxysilyl)propyl]hexadécyldiméthyl-ammonium, le chlorure de [3-(triméthoxysilyl)propyl]dodécyldiméthylammonium, le chlorure de [3-(triméthoxysilyl)propyl]octylammonium, la N-[3-(triméthoxysilyl)propyl]-aniline, le 3-[2-(2-aminoéthylamino)éthylamino]propyltriméthoxysilane, la N-[3-(triméthoxy-silyl)propyl]-N'-(4-vinylbenzyl)éthylènediamine, le triéthoxy-3-(2-imidazolin-1-yl)propyl-silane, la 1-[3-(triméthoxy-silyl)propyl]urée, la N-[3-(triméthoxysilyl)propyl]éthylènediamine, le [3-(diéthylamino)propyl]triméthoxysilane, le (3-glycidyloxypropyl)triméthoxysilane, le méthacrylate de 3-(triméthoxysilyl)propyle, le [2-(cyclohexényl)éthyl]triéthoxysilane, le dodécyltriéthoxysilane, l'hexadécyltriméthoxysilane, le (3-aminopropyl)triméthoxysilane, le (3-mercaptopropyl)triméthoxysilane, le (3-chloropropyl)triméthoxysilane, ainsi que les mélanges de deux ou plusieurs d'entre eux en toutes proportions.

Parmi les gabarits sacrificiels listés ci-dessus, on préfère tout particulièrement le chlorure de [3-(triméthoxy-silyl)propyl]octadecyldiméthylammonium, ou TPOAC.

On peut également utiliser des gabarits sacrificiels de masse molaire plus élevée et par exemple les PPDA (Polymer Poly-DiallyldimethylAmmonium), PVB (PolyVinyl Butyral) et autres composés oligomères connus dans le domaine pour augmenter le diamètre des mésopores.

De préférence on procède, à l'étape a), à l'agglomération d'éléments cristallins d'au moins une zéolithe FAU de haute surface externe, comme décrit précédemment, préparée en présence d'un gabarit sacrificiel destiné à être éliminé.

Cette élimination peut être réalisée selon les méthodes connues de l'homme du métier, par exemple par calcination, et de manière non limitative, la calcination des éléments cristallins de zéolithe comprenant le gabarit sacrificiel peut être effectuée sous balayage gazeux oxydant et/ou inerte, avec notamment des gaz tels que l'oxygène, l'azote, l'air, un air sec et/ou décarbonaté, un air appauvri en oxygène, éventuellement sec et/ou décarbonaté, à une ou des températures supérieures à 150°C, typiquement comprises entre 180°C et 800°C, préférentiellement entre 200°C et 650°C, pendant quelques heures, par exemple entre 2 et 6 heures. La nature des gaz, les rampes de montée en température et les paliers successifs de températures, leurs durées seront adaptées en fonction de la nature du gabarit sacrificiel.

L'étape supplémentaire d'élimination de l'éventuel gabarit sacrificiel peut être effectuée à tout moment au cours du procédé de préparation du matériau zéolithique aggloméré de l'invention. L'élimination dudit gabarit sacrificiel peut ainsi avantageusement être effectuée par calcination des éléments cristallins de zéolithe avant l'étape d'agglomération a), ou encore de manière concomitante avec la calcination des agglomérés lors de l'étape a).

La synthèse de zéolithe de type FAU se fait généralement en milieu sodique (cation Na). Les éléments cristallins de zéolithe FAU ainsi obtenus comprennent majoritairement, voir exclusivement des cations sodium. On ne sortirait toutefois pas du cadre de l'invention en utilisant des éléments cristallins ayant subi un ou plusieurs échanges cationiques, entre la synthèse sous forme Na, avant ou après l'élimination éventuelle du gabarit sacrificiel si cette étape est opérée avant la mise en oeuvre à l'étape a) et sa mise en oeuvre à l'étape a). Dans ce cas, l'étape c) et éventuellement l'étape d) d'échange devien(nen)t par conséquent non nécessaire(s).

La taille des éléments cristallins de zéolithe FAU utilisés à l'étape a) et des éléments cristallins de zéolithe FAU dans les agglomérés selon l'invention est mesurée par observation au microscope électronique à balayage (MEB). Comme indiqué précédemment, le diamètre moyen des éléments est compris entre 0,1 $\mu$m et 20 $\mu$m, de préférence entre 0,5 $\mu$m et 20 $\mu$m, de préférence encore entre 0,5 $\mu$m et 10 $\mu$m, et plus préférentiellement encore entre 0,5 $\mu$m et 5 $\mu$m bornes incluses. Cette observation MEB permet également de confirmer la présence de phase non zéolithique comprenant par exemple du liant résiduel (non converti lors de l'étape de zéolithisation) ou toute autre phase amorphe dans les agglomérés.

Dans le présent document, on emploie l'appellation « diamètre moyen en nombre » ou bien « taille », notamment pour les éléments cristallins de zéolithe et pour les adsorbants zéolithiques. La méthode de mesure de ces grandeurs est explicitée plus loin dans la description.

L'agglomération et la mise en forme (étape a) peuvent être réalisées selon toutes les techniques connues de l'homme de l'art, telles qu'extrusion, compactage, agglomération sur assiette granulatrice, tambour granulateur, atomisation et autres.

Les proportions de liant d'agglomération (voir définition plus loin) et de zéolithe mises en oeuvre sont typiquement celles de l'art antérieur, c'est-à-dire de 5 parties à 20 parties en poids de liant pour 95 parties à 80 parties en poids de zéolithe.

Les agglomérés issus de l'étape a), qu'ils soient sous forme de billes, d'extrudés ou autres, ont en général un diamètre moyen en nombre, ou leur longueur (plus grande dimension lorsqu'ils ne sont pas sphériques), comprise entre 0,2 mm et 2 mm, et en particulier comprise entre 0,2 mm et 0,8 mm et de préférence entre 0,4 mm et 0,65 mm, bornes incluses.

À l'issue de l'étape a) les agglomérés les plus fins peuvent être éliminés par cyclonage et/ou tamisage et/ou les agglomérés trop gros par tamisage ou concassage, dans le cas d'extrudés, par exemple.

Le liant compris dans le matériau aggloméré zéolithique de la présente invention comprend, et de préférence consiste en, une argile ou un mélange d'argiles. Ces argiles sont de préférence choisies parmi les kaolins, kaolinites, nacrites, dickites, halloysites, attapulgites, sépiolites, montmorillonites, bentonites, illites et métakaolins, ainsi que les mélanges de deux ou plusieurs d'entre elles en toutes proportions

Dans le cas de l'étape optionnelle b) de zéolithisation le liant d'agglomération mis en oeuvre à l'étape a) contient au moins 80 % de préférence, au moins 90%, de préférence encore au moins 95%, plus particulièrement au moins 96%, en poids, d'au moins une argile zéolithisable et peut également contenir d'autres liants minéraux tels que bentonite, attapulgite, et autres. Par argile zéolithisable, on entend une argile ou un mélange d'argiles qui sont susceptibles d'être converties en matière zéolithique, le plus souvent par action d'une solution basique alcaline. L'argile zéolithisable appartient en général à la famille des kaolins, kaolinites, nacrites, dickites, halloysite et/ou métakaolins. Le kaolin est préféré et le plus couramment utilisé.

Parmi les additifs éventuellement mis en oeuvre à l'étape a), on peut trouver une source de silice de tout type connu de l'homme du métier, spécialiste de la synthèse de zéolithes, par exemple de la silice colloïdale, des diatomées, de la perlite, des cendres de calcination (« fly ash » en langue anglaise), du sable, ou toute autre forme de silice solide.

Lors de l'étape a), outre les éléments cristallins de zéolithe FAU et le liant, d'autres additifs peuvent également être ajoutés, par exemple des additifs destinés à faciliter l'agglomération ou à améliorer le durcissement des agglomérés formés tels que la lignine, l'amidon, la carboxyméthylcellulose, et autres additifs connus de l'homme du métier.

Pour la calcination comprise dans l'étape a), la nature des gaz, les rampes de montée en température et les paliers successifs de températures, ainsi que leurs durées respectives, seront adaptés en fonction de la nature du gabarit sacrificiel à éliminer et en fonction de la nature du liant mis en oeuvre à l'étape d'agglomération a).

En particulier si le liant d'agglomération contient une ou plusieurs argiles zéolithisables, la calcination permet de transformer l'argile zéolithisable, typiquement le kaolin, en méta-kaolin qui peut après être converti en zéolithe lors de l'étape de zéolithisation (étape b)). Le principe en est exposé dans « Zeolite Molecular Sieves » de D. W. Breck, John Wiley and Sons, New York, (1973), p. 314-315.

La zéolithisation du liant d'agglomération est pratiquée selon toute méthode maintenant bien connue de l'homme du métier et peut par exemple être réalisée par immersion du produit issu de l'étape a) dans une solution basique alcaline, en général aqueuse, par exemple une solution aqueuse d'hydroxyde de sodium et/ou d'hydroxyde de potassium.

En règle générale, la concentration de la solution alcaline de zéolithisation est de préférence comprise entre 0,5 M et 5 M. La zéolithisation s'opère de préférence à chaud, à une température supérieure à la température ambiante, et typiquement à des températures de l'ordre de 80°C à 100°C, par exemple comprises entre la température ambiante (soit environ 20°C) et la température d'ébullition de la solution alcaline de zéolithisation. La durée du processus de zéolithisation est généralement comprise entre quelques dizaines de minutes et quelques heures, de préférence entre environ 1 heure et 8 heures.

Les étapes d'échange(s) cationique(s) c) et d) s'effectuent selon les méthodes classiques connues de l'homme du métier, et le plus souvent par mise en contact des agglomérés issus de l'étape a) avec un sel de baryum et/ou de potassium, tel que le chlorure de baryum ($BaCl_2$) et/ou de potassium (KCl), en solution aqueuse à une température comprise entre la température ambiante et 100°C, et de préférence comprise entre 80°C et 100°C afin d'obtenir rapidement des teneurs en baryum, exprimées en oxyde de baryum, élevées, i.e. de préférence supérieures à 10%, de préférence supérieures à 15%, de manière très préférée supérieures à 20%, de manière encore plus préférée supérieures à 23%, voire supérieures à 33% en poids par rapport à la masse totale de l'adsorbant.

Avantageusement, la teneur en baryum, exprimée en oxyde de baryum, est comprise entre 23% et 42%, et typiquement entre 30% et 40%, bornes incluses, en poids par rapport au poids total de l'adsorbant. On préfère opérer avec un large excès d'ions baryum par rapport aux cations de la zéolithe que l'on souhaite échanger, typiquement un excès de l'ordre de 10 à 12, avantageusement en procédant par échanges successifs.

L'échange éventuel au potassium (étape d) peut être pratiqué avant et/ou après l'échange au baryum (étape c). Comme indiqué précédemment, il est également possible d'agglomérer à l'étape a) des éléments cristallins de zéolithe FAU contenant déjà des ions baryum ou potassium ou baryum et potassium (pré-échange des cations présents dans la zéolithe de type FAU de départ, typiquement cations sodium, par des ions baryum ou potassium ou baryum et potassium avant l'étape a) et s'affranchir (ou non) des étapes c) et/ou d).

De manière surprenante, la demanderesse a observé que l'étape d'échange cationique, qui peut-être délicate en raison de la relative fragilité de la structure des éléments cristallins zéolithiques n'affecte pas les propriétés intrinsèques de surface externe et du volume microporeux (ramené à la masse de l'adsorbant une fois échangé) desdits éléments cristallins zéolithiques.

Après la ou les étape(s) d'échange(s) cationique(s), on procède ensuite à un lavage, généralement et de préférence à l'eau, puis d'un séchage de l'aggloméré ainsi obtenu.

L'activation qui suit le séchage, est conduite de manière classique, selon les méthodes connues de l'homme du métier, par exemple à une température en général comprise entre 100°C et 400°C, de préférence entre 200°C et 300°C pendant une durée déterminée en fonction de la teneur en eau et de la perte au feu souhaitées, typiquement de 1 heure à 6 heures.

La présente invention concerne également les utilisations des adsorbants zéolithiques décrits ci-dessus comme agents d'adsorption susceptibles de remplacer avantageusement les agents d'adsorption décrits dans la littérature, à base de cristaux conventionnels de zéolithe de type FAU, comprenant du baryum ou du baryum et du potassium dont les teneurs sont respectivement exprimées en teneurs en oxyde de baryum ou en oxyde de baryum et de potassium comme indiqué précédemment, ou à base de cristaux conventionnels de zéolithe de type FAU comprenant du baryum ou du baryum et du potassium, et notamment dans les utilisations listées ci-dessous :

- séparation de coupes d'isomères aromatiques en C8 et notamment des xylènes,
- séparation d'isomères de toluène substitué tels que nitrotoluène, diéthyltoluène, toluènediamine, et autres,
- séparation des crésols,
- la séparation des alcools polyhydriques, tels que les sucres.

L'invention concerne notamment un procédé de séparation de para-xylène à partir de coupes d'isomères aromatiques à 8 atomes de carbone, selon la revendication 12.

On peut ainsi séparer le produit désiré (para-xylène) par chromatographie liquide d'adsorption préparative (en batch), et avantageusement en continu en lit mobile simulé, c'est-à-dire à contre-courant simulé ou à co-courant simulé, et plus particulièrement à contre-courant simulé.

Les conditions opératoires d'une unité industrielle d'adsorption de type contre-courant simulé sont en général les suivantes :

- nombre de lits : 6 à 30,
- nombre de zones : au moins 4 zones de fonctionnement, chacune étant localisées entre un point d'alimentation et un point de soutirage,
- température comprise entre 100°C et 250°C, de préférence entre 150°C et 190°C,
- pression de l'unité industrielle comprise entre la pression de bulle des xylènes à la température du procédé et 3 MPa,
- rapport des débits désorbant/charge compris entre 0,7 et 2,5, par exemple entre 0,9 et 1,8 pour une unité d'adsorption seule (dite "stand alone") et entre 0,7 et 1,4 pour une unité d'adsorption combinée à une unité de cristallisation,

- taux de recyclage compris entre 2,5 et 12, de préférence entre 3,5 et 6.

On pourra sur ce sujet se référer à l'enseignement des brevets US2985589, US5284992 et US5629467.

Les conditions opératoires d'une unité industrielle d'adsorption à co-courant simulé sont en général les mêmes que celles fonctionnant à contre-courant simulé, à l'exception du taux de recyclage qui est en général compris entre 0,8 et 7. On pourra sur cet aspect se référer aux brevets US4402832 et US4498991.

Le solvant de désorption peut être tout désorbant connu de l'homme du métier et dont le point d'ébullition est inférieur à celui de la charge, tel que le toluène mais aussi un désorbant dont le point d'ébullition est supérieur à celui de la charge, tel que le *para*-diéthylbenzène (PDEB). La sélectivité des adsorbants selon l'invention pour l'adsorption du *para*-xylène contenu dans des coupes aromatiques en C8 est optimale lorsque leur perte au feu, mesurée à 900°C, est de préférence inférieure ou égale à 7,7%, de préférence comprise entre 0 et 7,7%, de manière très préférée entre 3,0% et 7,7%, de manière plus préférée entre 3,5% et 6,5% et de manière encore plus préférée entre 4,5% et 6%, bornes incluses.

Les adsorbants zéolithiques agglomérés selon la présente invention possèdent à la fois les caractéristiques des adsorbants zéolithiques conventionnels connus de l'art antérieur, notamment les propriétés mécaniques et de microporosité, les caractéristiques de transfert de matière global étant maximisées par rapport à des adsorbants zéolithiques à base de cristaux conventionnels.

En outre le procédé de préparation des adsorbants agglomérés zéolithiques à zéolithe(s) FAU de surface externe importante selon l'invention, est un procédé de mise en oeuvre aisée, rapide et économique et donc facilement industrialisable avec un minimum d'étapes.

Les exemples suivants permettent d'illustrer l'objet de l'invention, et sont fournis à titre indicatif seulement, sans toutefois être destinés en aucune façon à limiter les divers modes de réalisation de la présente invention.

Dans les exemples qui suivent, les propriétés physiques des agglomérés sont évaluées par les méthodes connues de l'homme du métier, dont les principales d'entre elles sont rappelées ci-dessous.

TECHNIQUES DE CARACTERISATION

**Granulométrie des particules** :

L'estimation du diamètre moyen en nombre des éléments (i.e. cristaux) de zéolithe de type FAU utilisées à l'étape a) et des éléments (i.e. cristaux) de zéolithe X contenus dans les agglomérés est réalisée par observation au microscope électronique à balayage (MEB).

Afin d'estimer la taille des particules (i.e. cristaux) de zéolithe sur les échantillons, on effectue un ensemble de clichés à un grossissement d'au moins 5000. On mesure ensuite le diamètre d'au moins 200 particules à l'aide d'un logiciel dédié, par exemple le logiciel Smile View de l'éditeur LoGraMi. La précision est de l'ordre de 3%.

**Analyse chimique des adsorbants zéolithiques - rapport Si/Al et taux d'échange** :

Une analyse chimique élémentaire du produit final obtenu à l'issue des étapes a) à f) décrites précédemment, peut être réalisée selon différentes techniques analytiques connues de l'homme du métier. Parmi ces techniques, on peut citer la technique d'analyse chimique par fluorescence de rayons X telle que décrite dans la norme NF EN ISO 12677 : 2011 sur un spectromètre dispersif en longueur d'onde (WDXRF), par exemple Tiger S8 de la société Bruker.

La fluorescence X est une technique spectrale non destructive exploitant la photoluminescence des atomes dans le domaine des rayons X, pour établir la composition élémentaire d'un échantillon. L'excitation des atomes généralement par un faisceau de rayons X ou par bombardement avec des électrons, génère des radiations spécifiques après retour à l'état fondamental de l'atome. Le spectre de fluorescence X a l'avantage de dépendre très peu de la combinaison chimique de l'élément, ce qui offre une détermination précise, à la fois quantitative et qualitative. On obtient de manière classique après étalonnage pour chaque oxyde une incertitude de mesure inférieure à 0,4% en poids.

Ces analyses chimiques élémentaires permettent à la fois de vérifier le rapport atomique Si/Al de la zéolithe utilisée au sein de l'aggloméré et le rapport atomique Si/Al du produit final obtenu à l'issue des étapes a) à f) décrites précédemment, et de vérifier la qualité de l'échange ionique décrit à l'étape c) et à l'étape optionnelle d). Dans la description de la présente invention, l'incertitude de mesure du rapport atomique Si/Al est de ± 5%.

La qualité de l'échange ionique est liée au nombre de mole d'oxyde de sodium, $Na_2O$, restant dans l'aggloméré zéolithique après échange. Plus précisément, le taux d'échange par les ions baryum est estimé en évaluant le rapport entre le nombre de moles d'oxyde de baryum, BaO, et le nombre de moles de l'ensemble (BaO + $Na_2O$). De même, le taux d'échange par les ions baryum et/ou potassium est estimé en évaluant le rapport entre le nombre de moles de l'ensemble oxyde de baryum + oxyde de potassium (BaO + $K_2O$) et le nombre de moles de l'ensemble (BaO + $K_2O$ + $Na_2O$). Il est à noter que les teneurs en différents oxydes sont donnés, en pourcentage en poids par rapport au poids total de l'adsorbant zéolithique anhydre.

**Granulométrie des adsorbants zéolithiques** :

La détermination du diamètre moyen en nombre des adsorbants zéolithiques obtenus à l'issus de l'étape a) d'agglomération et de mise en forme est effectuée par analyse de la distribution granulométrique d'un échantillon d'aggloméré par imagerie selon la norme ISO 13322-2:2006, en utilisant un tapis roulant permettant à l'échantillon de passer devant l'objectif de la caméra.

Le diamètre moyen en nombre est ensuite calculé à partir de la distribution granulométrique en appliquant la norme ISO 9276-2:2001. Dans le présent document, on emploie l'appellation « diamètre moyen en nombre » ou bien « taille » pour les agglomérés zéolithiques. La précision est de l'ordre de 0,01 mm pour la gamme de taille d'agglomérés de l'invention.

**Résistance mécanique des adsorbants zéolithiques :**

La résistance à l'écrasement d'un lit d'adsorbants zéolithiques tels que décrits dans la présente invention est caractérisée selon la méthode Shell série SMS1471-74 (Shell Method Series SMS1471-74 « Détermination of Bulk Crushing Strength of Catalysts. Compression-Sieve Method »), associée à l'appareil « BCS Tester » commercialisé par la société Vinci Technologies. Cette méthode, initialement destinée à la caractérisation de catalyseurs de 3 mm à 6 mm est basée sur l'utilisation d'un tamis de 425 $\mu$m qui va permettre notamment de séparer les fines créées lors de l'écrasement. L'utilisation d'un tamis de 425 $\mu$m reste adaptée pour des particules de diamètre supérieur à 1,6 mm, mais doit être adaptée selon la granulométrie des agglomérés que l'on cherche à caractériser.

Les agglomérés de la présente invention, généralement sous forme de billes ou d'extrudés, ont en général un diamètre moyen en nombre ou une longueur, i.e. plus grande dimension dans le cas des agglomérés non sphériques, comprise entre 0,2 mm et 2 mm, et en particulier comprise entre 0,2 mm et 0,8 mm et de préférence entre 0,4 mm et 0,65 mm, bornes incluses. Par conséquent, un tamis de 100 $\mu$m est utilisé à la place du tamis de 425 $\mu$m mentionné dans la méthode Shell standard SMS1471-74.

Le protocole de mesure est le suivant : un échantillon de 20 cm$^3$ d'adsorbants agglomérés, préalablement tamisé avec le tamis adapté (100 $\mu$m) et préalablement séché à l'étuve pendant au moins 2 heures à 250°C (au lieu de 300°C mentionné dans la méthode Shell standard SMS1471-74), est placé dans un cylindre métallique de section interne connue. Une force croissante est imposée par paliers sur cet échantillon par l'intermédiaire d'un piston, à travers un lit de 5 cm$^3$ de billes d'acier afin de mieux répartir la force exercée par le piston sur les agglomérés d'adsorbants (utilisation de billes de 2 mm de diamètre pour des particules de forme sphérique de diamètre strictement inférieur à 1,6 mm). Les fines obtenues aux différents paliers de pression sont séparées par tamisage (tamis adapté de 100 $\mu$m) et pesées.

La résistance à l'écrasement en lit est déterminée par la pression en mégaPascal (MPa) pour laquelle la quantité de fines cumulées passant à travers le tamis s'élève à 0,5% pondéral de l'échantillon. Cette valeur est obtenue en traçant sur un graphique la masse de fines obtenue en fonction de la force appliquée sur le lit d'adsorbant et en interpolant à 0,5 % massique de fines cumulées. La résistance mécanique à l'écrasement en lit est typiquement comprise entre quelques centaines de kPa et quelques dizaines de MPa et généralement comprise entre 0,3 MPa et 3,2 MPa. La précision est de manière classique inférieure à 0,1 MPa.

**Phase non zéolithique des adsorbants zéolithiques** :

Le taux de phase non zéolithique PNZ, par exemple liant résiduel non zéolithisé ou toute autre phase amorphe, après zéolithisation est calculé selon l'équation suivante :

$$PNZ = 100 - \Sigma\,(PZ),$$

où PZ représente la somme des quantités des fractions zéolithiques X au sens de l'invention.

La quantité des fractions zéolithiques X (taux de cristallinité) est mesurée par analyse par diffraction de rayons X, connue de l'homme du métier sous l'acronyme DRX. Cette analyse est réalisée sur un appareil de la marque Bruker, puis la quantité des fractions zéolithiques X est évaluée au moyen du logiciel TOPAS de la société Bruker.

**Volume microporeux et surface externe** :

La cristallinité des agglomérés est également évaluée par mesure de leur volume microporeux en le comparant à celui d'une référence appropriée (zéolithe 100% cristalline dans des conditions de traitements cationiques identiques ou zéolithe théorique). Ce volume microporeux est déterminé à partir de la mesure de l'isotherme d'adsorption de gaz, tel que l'azote, à sa température de liquéfaction.

Préalablement à l'adsorption, l'adsorbant zéolithique est dégazé entre 300°C et 450°C pendant une durée comprise entre 9 heures et 16 heures, sous vide (P < 6,7.10⁻⁴ Pa). La mesure de l'isotherme d'adsorption d'azote à 77 K est ensuite effectuée sur un appareil de type ASAP 2020 M de Micromeritics, en prenant au moins 35 points de mesure à des pressions relatives de rapport $P/P_0$ compris entre 0,002 et 1.

Le volume microporeux ainsi que la surface externe sont déterminés à partir de l'isotherme obtenue, par la méthode du t-plot en appliquant la norme ISO 15901-3:2007 et en calculant l'épaisseur statistique t par l'équation de Harkins-Jura. Le volume microporeux et la surface externe sont obtenus par régression linéaire sur les points du t-plot compris entre 0,45 nm et 0,7 nm, respectivement à partir de l'ordonnée à l'origine et de la pente de la régression linéaire. Le volume microporeux évalué s'exprime en cm³ d'adsorbat liquide par gramme d'adsorbant. La surface externe s'exprime en m² par gramme d'adsorbant.

**Volume total des macropores et des mésopores** :

Le volume total des macropores et des mésopores a été mesuré par porosimétrie par intrusion de mercure. La technique d'intrusion de mercure est utilisée pour caractériser le volume poreux intra-granulaire contenu dans les pores de diamètres supérieurs à 3,6 nm du matériau granulaire zéolithique, et pour mesurer sa densité granulaire. Un porosimètre à mercure type Autopore® 9500 de Microméritics est utilisé pour analyser la répartition du volume poreux contenu dans les macropores de diamètre de pore supérieur à 50 nm et dans les mésopores compris entre 3,6 nm et 50 nm. La fraction en volume des macropores est calculée en divisant le volume poreux contenu dans les macropores par le volume total des macropores et des mésopores.

La méthode expérimentale, décrite dans le manuel opératoire de l'appareil, consiste à placer un échantillon d'adsorbant (de perte au feu connue) préalablement pesé, dans une cellule du porosimètre, puis, après un dégazage préalable (pression d'évacuation de 30 μm Hg pendant au moins 10 min), à remplir la cellule avec du mercure à une pression donnée (0,0036 MPa), et ensuite à appliquer une pression croissante par palier jusqu'à 400 MPa afin de faire pénétrer progressivement le mercure dans le réseau poreux de l'échantillon.

La relation entre la pression appliquée et le diamètre des pores est établie en supposant des pores cylindriques, un angle de contact entre le mercure et la paroi des pores de 140° et une tension de surface du mercure de 485 dynes/cm.

**Perte au feu des adsorbants zéolithiques** :

La perte au feu est déterminée en atmosphère oxydante, par calcination de l'échantillon à l'air à une température de 900°C ± 25°C, en suivant le mode opératoire décrit dans la norme NF EN 196-2 (avril 2006). L'écart type de mesure est inférieur à 0,1%.

**Caractérisation de l'adsorption en phase liquide par perçage** :

La technique utilisée pour caractériser l'adsorption de molécules en phase liquide sur un solide poreux est la technique dite de perçage, décrite par Ruthven dans « Principles of Adsorption and Adsorption Processes » (John Wiley & Sons, (1984), Chapitres 8 et 9) qui définit la technique des courbes de perçage (« breakthrough curves ») comme l'étude de la réponse à l'injection d'un échelon de constituants adsorbables.

L'analyse du temps moyen de sortie (premier moment) des courbes de perçage fournit une information sur les quantités adsorbées et permet également d'évaluer les sélectivités, c'est-à-dire le facteur de séparation, entre deux constituants adsorbables. L'injection d'un constituant non adsorbable utilisé comme traceur est conseillée pour l'estimation des volumes non-sélectifs. L'analyse de la dispersion (second moment) des courbes de perçage, permet d'évaluer la hauteur équivalente de plateaux théoriques, basée sur la représentation d'une colonne par un nombre fini de réacteurs hypothétiques idéalement agités (étages théoriques), qui est une mesure directe de la dispersion axiale et de la résistance au transfert de matière du système.

**Exemple 1** : Synthèse de zéolithe FAU à haute surface externe.

La zéolithe FAU à haute surface externe est synthétisée de manière directe comme décrit dans l'article Inayat et coll., Angew. Chem. Int. Ed., (2012), 51, 1962-1965).

*Étape 1) : Préparation du gel de croissance dans réacteur agité avec vis d'Archimède à 300 tr.min⁻¹.*

Dans un réacteur en inox muni d'une double enveloppe chauffante, d'une sonde température et d'un agitateur, on prépare un gel de croissance en mélangeant une solution d'aluminate contenant 119 g d'hydroxyde de sodium (NaOH) à, 128 g d'alumine trihydratée ($Al_2O_3$, $3H_2O$, contenant 65,2% en poids d'$Al_2O_3$) et 195,5 g eau à 25°C en 25 minutes avec une vitesse d'agitation de 300 tr.min$^{-1}$ dans une solution de silicate contenant 565,3 g de silicate de sodium, 55,3 g de NaOH et 1997,5 g d'eau à 25°C.

La stœchiométrie du gel de croissance est la suivante : 3,48 $Na_2O$ / $Al_2O_3$ / 3,07 $SiO_2$ / 180 $H_2O$. L'homogénéisation du gel de croissance est réalisée sous agitation à 300 tr.min$^{-1}$, pendant 25 minutes à 25°C.

### *Étape 2) : Introduction dans le milieu réactionnel de l'agent structurant*

On introduit dans le milieu réactionnel 27,3 g de solution de TPOAC à 60% dans le MeOH avec une vitesse d'agitation de 300 tr.min$^{-1}$ (ratio molaire TPOAC/$Al_2O_3$ = 0,04). Après 5 minutes d'homogénéisation, on baisse la vitesse d'agitation à 50 tr.min$^{-1}$.

### *Étape 3) : Phase de mûrissement*

On maintient le milieu réactionnel agité à 50 tr.min$^{-1}$ à 25°C, pendant 22 heures, puis on démarre la cristallisation.

### *Étape 4) : Cristallisation*

On maintient la vitesse d'agitation à 50 tr.min$^{-1}$, et on fixe la consigne de la double enveloppe du réacteur à 80°C afin que le milieu réactionnel monte en température à 75°C en 80 minutes. Après 72 heures de palier à 75°C, on refroidit le milieu réactionnel en faisant circuler de l'eau froide dans la double enveloppe pour stopper la cristallisation.

### *Étape 5) : Filtration / lavage*

Les solides sont récupérés sur fritté puis lavés avec de l'eau permutée jusqu'à pH neutre.

### *Étape 6)* : **Séchage / Calcination**

Afin de caractériser le produit, le séchage est réalisé en étuve à 90°C pendant 8 heures, la perte au feu du produit séché est de 22% en poids.

La calcination du produit séché nécessaire pour libérer à la fois la microporosité (eau) et la mésoporosité en éliminant l'agent structurant est effectuée avec le profil de température suivant : 30 minutes de montée à 200°C, puis 1 heure de palier à 200°C, puis 3 heures de montée à 550°C, et enfin 1,5 heures de palier à 550°C.

Le volume microporeux et la surface externe mesurés selon la méthode du t-plot à partir de l'isotherme d'adsorption d'azote à 77 K après dégazage sous vide à 400°C pendant 10 heures sont respectivement 0,260 cm$^3$.g$^{-1}$ et 90 m$^2$.g$^{-1}$. Le diamètre moyen en nombre des éléments cristallins est de 4,5 $\mu$m.

Dans ce qui suit une masse exprimée en équivalent anhydre signifie une masse de produit diminuée de sa perte au feu.

### **Exemple 2** : (comparatif)

On prépare un mélange homogène constitué de 1600 g équivalent anhydre de cristaux de zéolithe X de diamètre moyen en nombre égal à 1,0 $\mu$m, de 350 g équivalent anhydre de kaolin, de 130 g de silice colloïdale vendue sous la dénomination commerciale de Klebosol® 30 (contenant 30% en poids de $SiO_2$ et 0,5% de $Na_2O$), ainsi que de la quantité d'eau qui permet l'extrusion du mélange. La perte au feu de la pâte avant extrusion est de 44%.

On forme des extrudés de 1,6 mm de diamètre. Les extrudés sont séchés une nuit en étuve ventilée à 80°C. Ils sont ensuite calcinés pendant 2 heures à 550°C sous balayage à l'azote, puis 2 heures à 550°C sous balayage à l'air sec décarbonaté puis concassés de manière à récupérer des grains dont le diamètre équivalent est égal à 0,4 mm.

Ces granulés sont échangés au moyen d'une solution de chlorure de baryum 0,5 M à 95°C en 4 étapes. À chaque étape, le ratio volume de solution sur masse de solide est de 20 mL.g$^{-1}$ et l'échange est poursuivi pendant 4 heures à chaque fois. Entre chaque échange, le solide est lavé plusieurs fois de manière à le débarrasser des excédents de sel. Il est ensuite activé à une température de 250°C pendant 2 heures sous courant d'azote.

Le taux d'échange en baryum est de 97% et la perte au feu (mesurée à 900°C) est de 5,4%. Le volume microporeux et la surface externe mesurés selon la méthode du t-plot à partir de l'isotherme d'adsorption d'azote à 77 K après dégazage sous vide à 400°C pendant 10 heures sont respectivement 0,226 cm$^3$.g$^{-1}$ et 16,7 m$^2$.g$^{-1}$.

Le volume total des macropores et des mésopores mesuré par porosimétrie mercure est de 0,32 cm$^3$.g$^{-1}$. La fraction de volume des macropores sur le volume total des macropores et mésopores est égale à 0,87.

Un test de perçage (chromatographie frontale) est ensuite réalisé sur ces adsorbants pour évaluer leur efficacité. La quantité d'adsorbant utilisée pour ce test est d'environ 34 g.

Le mode opératoire pour obtenir les courbes de perçage est le suivant :

- Remplissage de la colonne par le tamis et mise en place dans le banc de test.
- Remplissage par le solvant à température ambiante.
- Montée progressive à la température d'adsorption sous flux de solvant (5 cm$^3$/min).
- Injection de solvant à 30 cm$^3$/min lorsque la température d'adsorption est atteinte.
- Permutation solvant/charge pour injecter la charge (30 cm$^3$.min$^{-1}$).
- L'injection de la charge est ensuite maintenue un temps suffisant pour atteindre l'équilibre thermodynamique (c'est-à-dire jusqu'à ce que la concentration en solvant dans l'effluent soit nulle).
- Collecte et analyse de l'effluent du perçage.

Le solvant utilisé est le para-diéthylbenzène. La composition de la charge est la suivante :

Paraxylène : 45% poids
Métaxylène : 45% poids
Iso-octane : 10% poids (celui-ci est utilisé comme traceur pour l'estimation des volumes non-sélectifs et n'intervient pas dans la séparation)

Un premier test est réalisé avec une température d'adsorption de 175°C et un second test est réalisé avec une température d'adsorption de 160°C. La pression est suffisante pour que la charge reste en phase liquide, soit 1 MPa.

La sélectivité du paraxylène par rapport au *méta*-xylène est calculée par bilan matière. Les résultats du perçage sont consignés dans le Tableau 1 ci-dessous :

**-- Tableau 1 --**

| T(°C) | sélectivité PX/MX | Capacité d'adsorption (cm$^3$.g$^{-1}$) | Vitesse fût vide (cm.s$^{-1}$) | HEPT PX (cm) |
|---|---|---|---|---|
| 175 | 3,35 | 0,191 | 1,32 | 6,31 |
| 160 | 3,72 | 0,189 | 1,29 | 19,23 |

Légende

- PAF = Perte Au Feu
- T (°C) = température d'adsorption
- Capacité d'adsorption exprimée en cm$^3$ de Cs-aromatiques adsorbés par gramme d'adsorbant
- Vitesse fût vide = vitesse superficielle (débit/section de la colonne)
- HEPT = Hauteur Équivalente de Plateaux Théoriques (en cm)
- PX = ParaXylène ; MX = MétaXylène

On mesure également la résistance mécanique selon la méthode présentée dans la description de l'invention. La pression nécessaire pour obtenir 0,5% de fines est de 2,2 MPa.

**Exemple 2 bis :** (comparatif)

Dans cet exemple, on réalise et on teste un adsorbant selon l'art antérieur (FR2789914). L'exemple 2 de FR2789914 a été reproduit à l'identique en utilisant des cristaux de zéolithe NaX industrielle (poudre de dénomination commerciale G5, de rapports atomiques Si/Al = 1,25 et Na/Al = 1, et de diamètre moyen en nombre égal à 2,1 μm et de la silice colloïdale vendue sous la dénomination commerciale de Klebosol® 30 (commercialisée antérieurement sous la dénomination Cecasol® 30).

L'adsorbant ainsi préparé selon l'exemple 2 de FR2789914 présente un taux d'échange en baryum de 97,4% et une perte au feu (mesurée à 900°C) de 5,2%. Le volume microporeux et la surface externe sont mesurés à partir de l'isotherme d'adsorption d'azote à 77 K après dégazage sous vide à 400°C pendant 10 heures. Le volume microporeux mesuré selon la méthode de Dubinin-Radushkevitch et celui mesuré selon la méthode du t-plot sont identiques et égaux à 0,248 cm$^3$.g$^{-1}$ et la surface externe mesurée selon la méthode du t-plot est de 2 m$^2$.g$^{-1}$.

Le volume total des macropores et des mésopores mesuré par porosimétrie mercure est de 0,304 cm$^3$.g$^{-1}$. La fraction de volume des macropores sur le volume total des macropores et mésopores est égale à 0,94.

Un test de perçage (chromatographie frontale) est ensuite réalisé sur ces adsorbants pour évaluer leur efficacité. La quantité d'adsorbant utilisée pour ce test est d'environ 35 g. Le test est réalisé uniquement à une température d'adsorption de 175°C.

La composition de la charge et le mode opératoire pour obtenir les courbes de perçage sont identiques à ce qui décrit dans l'exemple 2. La sélectivité du paraxylène par rapport au métaxylène est calculée par bilan matière. Les résultats du perçage sont consignés dans le Tableau 2 ci-dessous :

-- Tableau 2 --

| T (°C) | sélectivité PX/MX | Capacité d'adsorption (cm$^3$.g$^{-1}$) | Vitesse fût vide (cm.s$^{-1}$) | HEPTPX (cm) |
|---|---|---|---|---|
| 175 | 3,60 | 0,205 | 1,3 | 38,1 |

Légende

- T (°C) = température d'adsorption
- Capacité d'adsorption exprimée en cm$^3$ de Cs-aromatiques adsorbés par gramme d'adsorbant
- Vitesse fût vide = vitesse superficielle (débit/section de la colonne)
- HEPT = Hauteur Équivalente de Plateaux Théoriques (en cm)
- PX = ParaXylène ; MX = MétaXylène

**Exemple 2 ter :** (comparatif)

Dans cet exemple, on réalise et on teste un adsorbant selon l'art antérieur. L'exemple 3 de FR2903978 comparatif selon l'art antérieur FR2789914 est reproduit à l'identique en utilisant des cristaux de zéolithe NaX industrielle ayant un rapport atomique Si/Al égal à 1,25, un rapport atomique Na/Al égal à 1, et un diamètre moyen en nombre des cristaux mesuré égal à 2,1 μm, et de la silice colloïdale vendue sous la dénomination commerciale de Klebosol® 30 (antérieurement sous la dénomination Cecasol® 30).

L'adsorbant ainsi préparé présente un taux d'échange en baryum de 95% et une perte au feu (mesurée à 900°C) de 6,0%. Le volume microporeux et la surface externe sont mesurés à partir de l'isotherme d'adsorption d'azote à 77 K après dégazage sous vide à 400°C pendant 10 heures. Le volume microporeux mesuré selon la méthode de Dubinin-Radushkevitch et celui mesuré selon la méthode du t-plot sont identiques et égaux à 0,252 cm$^3$.g$^{-1}$ et la surface externe mesurée selon la méthode du t-plot est de 3 m$^2$.g$^{-1}$.

Le volume total des macropores et des mésopores mesuré par porosimétrie mercure est de 0,280 cm$^3$.g$^{-1}$. La fraction de volume des macropores sur le volume total des macropores et mésopores est égale à 0,93.

Un test de perçage (chromatographie frontale) est ensuite réalisé sur ces adsorbants pour évaluer leur efficacité. La quantité d'adsorbant utilisée pour ce test est d'environ 35 g.

La composition de la charge et le mode opératoire pour obtenir les courbes de perçage sont identiques à ce qui décrit dans l'exemple 2. Le test est réalisé uniquement à une température d'adsorption de 175°C.

La sélectivité du para-xylène par rapport au *méta*-xylène est calculée par bilan matière. Les résultats du perçage sont consignés dans le Tableau 3 ci-dessous :

-- Tableau 3 --

| T(°C) | sélectivité PX/MX | Capacité d'adsorption (cm$^3$.g$^{-1}$) | Vitesse fût vide (cm.s$^{-1}$) | HEPTPX (cm) |
|---|---|---|---|---|
| 175 | 3,41 | 0,199 | 1,3 | 17,6 |

Légende

- T (°C) = température d'adsorption
- Capacité d'adsorption exprimée en cm$^3$ de Cs-aromatiques adsorbés par gramme d'adsorbant
- Vitesse fût vide = vitesse superficielle (débit/section de la colonne)

- HEPT = Hauteur Équivalente de Plateaux Théoriques (en cm)
- PX = ParaXylène ; MX = MétaXylène

On mesure également la résistance mécanique selon la méthode présentée dans la description de l'invention. La pression nécessaire pour obtenir 0,5% de fines est de 2,6 MPa.

<u>Exemple 3</u> : (selon l'invention)

On prépare de la même façon que dans l'exemple 2 un mélange homogène constitué de 1600 g équivalent anhydre de cristaux de zéolithe X synthétisée selon le mode opératoire de l'exemple 1, de 350 g équivalent anhydre de kaolin, de 130 g de silice colloïdale vendue sous la dénomination commerciale de Klebosol® 30 (contenant 30% en poids de $SiO_2$ et 0,5% de $Na_2O$) ainsi que de la quantité d'eau qui permet l'extrusion du mélange. La perte au feu de la pâte avant extrusion est de 44%.

On forme des extrudés de 1,6 mm de diamètre. Les extrudés sont séchés une nuit en étuve ventilée à 80°C. Ils sont ensuite calcinés pendant 2 heures à 550°C sous balayage à l'azote, puis 2 heures à 550°C sous balayage à l'air sec décarbonaté puis concassés de manière à récupérer des grains dont le diamètre équivalent est égal à 0,4 mm.

L'échange au baryum est opéré dans des conditions opératoires identiques à celles de l'exemple 2, à l'exception de la concentration de la solution de $BaCl_2$ qui est de 0,7 M suivi d'un lavage puis d'un séchage à 80°C pendant 2 heures et enfin d'une activation à 250°C pendant 2 heures sous courant d'azote.

Le taux d'échange en baryum est de 97% et la perte au feu (mesurée à 900°C) est de 5,5 %. Le volume microporeux et la surface externe mesurés selon la méthode du t-plot à partir de l'isotherme d'adsorption d'azote à 77 K après dégazage sous vide à 400°C pendant 10 heures sont respectivement 0,192 $cm^3.g^{-1}$ et 70 $m^2.g^{-1}$.

Le volume total des macropores et mésopores mesuré par porosimétrie mercure est de 0,33 $cm^3.g^{-1}$. La fraction de volume des macropores sur le volume total des macropores et mésopores est égale à 0,6.

Un test de perçage (chromatographie frontale) est ensuite réalisé sur ces adsorbants pour évaluer leur efficacité. La quantité d'adsorbant utilisée pour ce test est d'environ 34 g.

Le mode opératoire ainsi que la composition de la charge sont identiques à ceux de l'exemple 2. La sélectivité du para-xylène par rapport au *méta*-xylène est calculée par bilan matière. Les résultats du perçage sont réunis dans le Tableau 4 ci-dessous :

-- **Tableau 4** --

| T (°C) | sélectivité PX/MX | Capacité d'adsorption (cm³.g⁻¹) | Vitesse fût vide (cm/s) | HEPTPX (cm) |
|--------|-------------------|----------------------------------|---------------------------|--------------|
| 175 | 2,66 | 0,180 | 1,32 | 2,64 |
| 160 | 2,78 | 0,179 | 1,29 | 3,35 |

<u>Légende</u>

- T (°C) = température d'adsorption
- Capacité d'adsorption exprimée en $cm^3$ de Cs-aromatiques adsorbés par gramme d'adsorbant
- Vitesse fût vide = vitesse superficielle (débit/section de la colonne)
- HEPT = Hauteur Équivalente de Plateaux Théoriques (en cm)
- PX = ParaXylène ; MX = MétaXylène

On mesure également la résistance mécanique selon la méthode présentée dans la description de l'invention. La pression nécessaire pour obtenir 0,5% de fines est de 2,1 MPa.

Par rapport aux résultats obtenus avec l'adsorbant des exemples 2, 2bis et 2ter, on constate une nette amélioration du transfert de matière, la hauteur équivalente de plateaux théoriques ayant considérablement diminuée.

**Revendications**

**1.** Adsorbant zéolithique comprenant au moins une zéolithe FAU et comprenant du baryum et/ou du potassium, **caractérisé en ce que** la surface externe dudit adsorbant zéolithique, mesurée par adsorption d'azote, est supérieure à 20 $m^2.g^{-1}$, de préférence supérieure à 40 $m^2.g^{-1}$, et de préférence encore comprise entre 40 $m^2.g^{-1}$ et 200 $m^2.g^-$

1, et plus préférentiellement comprise entre 60 m$^2$.g$^{-1}$ et 200 m$^2$.g-1 bornes incluses, la surface externe étant déterminée à partir de l'isotherme d'adsorption d'azote obtenue par la méthode du t-plot en appliquant la norme ISO 15901-3 :2007, préalablement à l'adsorption, l'adsorbant zéolithique étant dégazé entre 300°C et 450°C pendant une durée comprise entre 9 heures et 16 heures, sous vide (P < 6,7.10$^{-4}$ Pa), et le diamètre moyen en nombre des éléments cristallins (ou « cristaux ») étant compris entre 0,1 $\mu$m et 20 $\mu$m.

2. Adsorbant zéolithique selon la revendication 1, dans lequel la teneur en oxyde de baryum (BaO) est supérieure à 10%, de préférence supérieure à 15%, de manière très préférée supérieure à 20%, de manière encore plus préférée supérieure à 23 %, voire supérieure à 33% en poids par rapport à la masse totale de l'adsorbant, et avantageusement, la teneur en baryum est comprise entre 23% et 42%, et typiquement entre 30% et 40%, bornes incluses, en poids par rapport au poids total de l'adsorbant, la teneur en oxyde de barium étant calculée à partir de l'analyse élémentaire de l'adsorbant, l'analyse élémentaire étant réalisée par fluorescence de rayons X telle que décrite dans la norme NF EN ISO 12677 : 2011.

3. Adsorbant zéolithique selon la revendication 1 ou la revendication 2, dans lequel la teneur en oxyde de potassium K$_2$O est inférieure à 25%, de préférence comprise entre 0 et 20%, de manière encore plus préférée comprise entre 0% et 15% et de manière très préférée de 0% à 10% bornes incluses en poids par rapport à la masse totale de l'adsorbant, la teneur en oxyde de potassium étant calculée à partir de l'analyse élémentaire de l'adsorbant, l'analyse élémentaire étant réalisée par fluorescence de rayons X telle que décrite dans la norme NF EN ISO 12677 : 2011.

4. Adsorbant selon l'une quelconque des revendications 1 à 3, dans lequel ladite zéolithe FAU présente un rapport atomique Si/Al compris entre 1,00 et 1,50 bornes incluses, de préférence entre 1,05 et 1,50, de préférence entre 1,05 et 1,40, bornes incluses et de manière encore plus préférée entre 1,10 et 1,40 bornes incluses, le rapport atomique Si/Al étant calculé à partir de l'analyse élémentaire de l'adsorbant, l'analyse élémentaire étant réalisé par fluorescence de rayons X telle que décrite dans la norme NF EN ISO 12677 : 2011.

5. Adsorbant selon l'une quelconque des revendications précédentes, dans lequel aucune structure zéolithique autre que la structure faujasite, de préférence aucune structure zéolithique autre que la structure faujasite X, n'est détectée par diffraction des rayons X.

6. Adsorbant selon l'une quelconque des revendications précédentes, dans lequel la fraction massique de zéolithe FAU, la zéolithe FAU étant de préférence une zéolithe X, est supérieure ou égale à 80% par rapport au poids total d'adsorbant.

7. Adsorbant selon l'une quelconque des revendications précédentes, dans lequel le volume total des macropores et des mésopores, mesuré par intrusion de mercure, est compris entre 0,15 cm$^3$.g$^{-1}$ et 0,5 cm$^3$.g$^{-1}$, de préférence compris entre 0,20 cm$^3$.g$^{-1}$ et 0,40 cm$^3$.g$^{-1}$ et de manière très préférée compris entre 0,20 cm$^3$.g$^{-1}$ et 0,35 cm$^3$.g$^1$, le volume total des macropores et des mésopores étant mesuré par porosimétrie par intrusion de mercure, l'échantillon d'adsorbant étant préalablement pesé, dans une cellule du porosimètre, puis, après un dégazage préalable à une pression d'évacuation de 30 $\mu$m Hg pendant au moins 10 min, la cellule est remplie avec du mercure à une pression de 0,0036 MPa, et ensuite une pression croissante est appliquée par palier jusqu'à 400 MPa afin de faire pénétrer progressivement le mercure dans le réseau poreux de l'échantillon, la relation entre la pression appliquée et le diamètre des pores étant établie en supposant des pores cylindriques, un angle de contact entre le mercure et la paroi des pores de 140° et une tension de surface du mercure de 485 dynes/cm

8. Adsorbant selon l'une quelconque des revendications précédentes, dans lequel la fraction en volume des macropores est comprise entre 0,2 et 1 du volume total des macropores et des mésopores, de manière très préférée comprise entre 0,4 et 0,8, et de manière encore plus préférée entre 0,45 et 0,65, bornes incluses, le volume total des macropores et des mésopores étant mesuré par porosimétrie par intrusion de mercure, l'échantillon d'adsorbant étant préalablement pesé, dans une cellule du porosimètre, puis, après un dégazage préalable à une pression d'évacuation de 30 $\mu$m Hg pendant au moins 10 min, la cellule est remplie avec du mercure à une pression de 0,0036 MPa, et ensuite une pression croissante est appliquée par palier jusqu'à 400 MPa afin de faire pénétrer progressivement le mercure dans le réseau poreux de l'échantillon, la relation entre la pression appliquée et le diamètre des pores étant établie en supposant des pores cylindriques, un angle de contact entre le mercure et la paroi des pores de 140° et une tension de surface du mercure de 485 dynes/cm

9. Adsorbant selon l'une quelconque des revendications précédentes, dont le diamètre moyen en nombre des éléments cristallins (ou « cristaux ») est compris entre 0,5 $\mu$m et 20 $\mu$m, de préférence encore entre 0,5 $\mu$m et 10 $\mu$m, et plus

préférentiellement encore entre 0,5 $\mu$m et 5 $\mu$m, bornes incluses.

10. Utilisation d'un adsorbant selon l'une quelconque des revendications 1 à 9, dans les procédés de :

- séparation de coupes d'isomères aromatiques en C8 et notamment des xylènes,
- séparation d'isomères de toluène substitué tels que nitrotoluène, diéthyltoluène, toluènediamine, et autres,
- séparation des crésols,
- séparation des alcools polyhydriques.

11. Utilisation selon la revendication 10, pour la séparation de para-xylène à partir de coupes d'isomères aromatiques à 8 atomes de carbone.

12. Procédé de séparation de para-xylène à partir de coupes d'isomères aromatiques à 8 atomes de carbone, utilisant comme agent d'adsorption du para-xylène, un adsorbant zéolithique comprenant du baryum et/ou du potassium présentant une surface externe **caractérisée par** adsorption d'azote, supérieure à 20 m$^2$.g$^{-1}$ , de préférence supérieure à 40 m$^2$.g$^{-1}$ , et de préférence encore comprise entre 40 m$^2$.g$^{-1}$ et 200 m$^2$.g$^{-1}$ , et plus préférentiellement comprise entre 60 m$^2$.g$^{-1}$ et 200 m$^2$.g$^{-1}$ bornes incluses, mis en œuvre dans des procédés en phase liquide, mais aussi en phase gazeuse, la surface externe de l'adsorbant étant déterminée à partir de l'isotherme d'adsorption d'azote obtenue par la méthode du t-plot en appliquant la norme ISO 15901-3 :2007, préalablement à l'adsorption, l'adsorbant zéolithique étant dégazé entre 300°C et 450°C pendant une durée comprise entre 9 heures et 16 heures, sous vide (P < 6,7.10$^{-4}$ Pa),
et le diamètre moyen en nombre des éléments cristallins (ou « cristaux ») étant compris entre 0,1 nm et 20 $\mu$m.

13. Procédé selon la revendication 12, utilisant comme agent d'adsorption du para- xylène, un adsorbant zéolithique selon l'une quelconque des revendications 1 à 9.

14. Procédé selon la revendication 12 ou la revendication 13, de séparation de para-xylène à partir de coupes d'isomères d'hydrocarbures aromatiques contenant 8 atomes de carbone, en phase liquide, par adsorption du para-xylène au moyen d'un adsorbant selon l'une quelconque des revendications 1 à 9, en présence d'un désorbant, de préférence choisi parmi le toluène et le *para-diéthylbenzène.*

**Patentansprüche**

1. Zeolithisches Adsorptionsmittel, umfassend mindestens einen FAU-Zeolith und umfassend Barium und/oder Kalium, **dadurch gekennzeichnet, dass** die äußere Oberfläche des zeolithischen Adsorptionsmittels, gemessen durch Stickstoffadsorption, größer als 20 m$^2$.g$^{-1}$, vorzugsweise größer als 40 m$^2$.g$^{-1}$ ist und vorzugsweise noch zwischen 40 m$^2$.g$^{-1}$ und 200 m$^2$.g$^{-1}$ liegt und vorzugsweiser zwischen 60 m$^2$.g$^{-1}$ und 200 m$^2$.g$^{-1}$ liegt, Grenzwerte inbegriffen, wobei die äußere Oberfläche ausgehend von der Stickstoffadsorptionsisotherme, erhalten durch die t-Plot-Methode bei Anwendung der Norm ISO 15901-3:2007 vor der Adsorption, bestimmt wird, wobei das zeolithische Adsorptionsmittel zwischen 300 °C und 450 °C während einer Dauer, die zwischen 9 Stunden und 16 Stunden liegt, im Vakuum (P < 6,7.10$^{-4}$ Pa) entgast wird,
und der zahlenmäßig mittlere Durchmesser der kristallinen Elemente (oder "Kristalle") zwischen 0,1 $\mu$m und 20 $\mu$m liegt.

2. Zeolithisches Adsorptionsmittel nach Anspruch 1, wobei der Gehalt an Bariumoxid (BaO) größer als 10 %, vorzugsweise größer als 15 %, sehr bevorzugt größer als 20 %, noch bevorzugter größer als 23 %, ja sogar größer als 33 % in Gewicht im Verhältnis zur Gesamtmasse des Adsorptionsmittels ist und in vorteilhafter Weise der Gehalt an Barium zwischen 23 % und 42 % und in typischer Weise zwischen 30 % und 40 %, Grenzwerte inbegriffen, in Gewicht im Verhältnis zum Gesamtgewicht des Adsorptionsmittels liegt, wobei der Gehalt an Bariumoxid ausgehend von der Elementaranalyse des Adsorptionsmittels berechnet wird, wobei die Elementaranalyse durch Röntgenstrahl-Fluoreszenz durchgeführt wird, so wie in der Norm NF EN ISO 12677:2011 beschrieben.

3. Zeolithisches Adsorptionsmittel nach Anspruch 1 oder Anspruch 2, wobei der Gehalt an Kaliumoxid $K_2O$ kleiner als 25 % ist, vorzugsweise zwischen 0 und 20 %, noch bevorzugter zwischen 0 % und 15 % und sehr bevorzugt von 0 % bis 10 %, Grenzwerte inbegriffen, in Gewicht im Verhältnis zur Gesamtmasse des Adsorptionsmittels liegt, wobei der Gehalt an Kaliumoxid ausgehend von der Elementaranalyse des Adsorptionsmittels berechnet wird, wobei die Elementaranalyse durch Röntgenstrahl-Fluoreszenz durchgeführt wird, so wie in der Norm NF EN ISO 12677:2011 beschrieben.

4. Adsorptionsmittel nach einem der Ansprüche 1 bis 3, wobei der FAU-Zeolith ein Atomverhältnis Si/Al aufweist, das zwischen 1,00 und 1,50, Grenzwerte inbegriffen, vorzugsweise zwischen 1,05 und 1,50, vorzugsweise zwischen 1,05 und 1,40, Grenzwerte inbegriffen und noch bevorzugter zwischen 1,10 und 1,40, Grenzwerte inbegriffen, liegt, wobei das Atomverhältnis Si/Al ausgehend von der Elementaranalyse des Adsorptionsmittels berechnet wird, wobei die Elementaranalyse durch Röntgenstrahl-Fluoreszenz durchgeführt wird, so wie in der Norm NF EN ISO 12677:2011 beschrieben.

5. Adsorptionsmittel nach einem der vorangehenden Ansprüche, wobei keine andere Zeolithstruktur als die Faujasitstruktur, vorzugsweise keine andere Zeolithstruktur als die X-Faujasitstruktur, durch Röntgenstrahldiffraktion ermittelt wird.

6. Adsorptionsmittel nach einem der vorangehenden Ansprüche, wobei der Massenanteil von FAU-Zeolith, wobei der FAU-Zeolith vorzugsweise ein X-Zeolith ist, größer oder gleich 80 % im Verhältnis zum Gesamtgewicht des Adsorptionsmittels ist.

7. Adsorptionsmittels nach einem der vorangehenden Ansprüche, wobei das Gesamtvolumen der Makroporen und der Mesoporen, gemessen durch Quecksilberintrusion, zwischen 0,15 $cm^3.g^{-1}$ und 0,5 $cm^3.g^{-1}$, vorzugsweise zwischen 0,20 $cm^3.g^{-1}$ und 0,40 $cm^3.g^{-1}$ und sehr bevorzugt zwischen 0,20 $cm^3.g^{-1}$ und 0,35 $cm^3.g^{-1}$ liegt, wobei das Gesamtvolumen der Makroporen und der Mesoporen durch Porosimetrie durch Quecksilberintrusion gemessen wird, wobei die Adsorptionsmittelprobe zuvor in einer Zelle des Porosimeters gewogen wird, dann, nach einer vorherigen Entgasung bei einem Ausleitungsdruck von 30 $\mu$m Hg während mindestens 10 min, die Zelle mit Quecksilber bei einem Druck von 0,0036 MPa gefüllt wird und danach ein stufenweise bis auf 400 MPa zunehmender Druck angewendet wird, damit das Quecksilber nach und nach in das Porennetz der Probe eindringt, wobei das Verhältnis zwischen dem angewendeten Druck und dem Durchmesser der Poren bei Annahme zylindrischer Poren, einem Kontaktwinkel zwischen dem Quecksilber und der Porenwand von 140° und einer Oberflächenspannung des Quecksilbers von 485 Dyne/cm ermittelt wird.

8. Adsorptionsmittels nach einem der vorangehenden Ansprüche, wobei der Anteil in Volumen der Makroporen zwischen 0,2 und 1 des Gesamtvolumens der Makroporen und der Mesoporen, sehr bevorzugt zwischen 0,4 und 0,8 und noch bevorzugter zwischen 0,45 und 0,65 liegt, Grenzwerte inbegriffen, wobei das Gesamtvolumen der Makroporen und der Mesoporen durch Porosimetrie durch Quecksilberintrusion gemessen wird, wobei die Adsorptionsmittelprobe zuvor in einer Zelle des Porosimeters gewogen wird, dann, nach einer vorherigen Entgasung bei einem Ausleitungsdruck von 30 $\mu$m Hg während mindestens 10 min, die Zelle mit Quecksilber bei einem Druck von 0,0036 MPa gefüllt wird und danach ein stufenweise bis auf 400 MPa zunehmender Druck angewendet wird, damit das Quecksilber nach und nach in das Porennetz der Probe eindringt, wobei das Verhältnis zwischen dem angewendeten Druck und dem Durchmesser der Poren bei Annahme zylindrischer Poren, einem Kontaktwinkel zwischen dem Quecksilber und der Porenwand von 140° und einer Oberflächenspannung des Quecksilbers von 485 Dyne/cm ermittelt wird.

9. Adsorptionsmittel nach einem der vorangehenden Ansprüche, dessen zahlenmäßig mittlerer Durchmesser der kristallinen Elemente (oder "Kristalle") zwischen 0,5 $\mu$m und 20 $\mu$m, vorzugsweise noch zwischen 0,5 $\mu$m und 10 $\mu$m und noch vorzugsweiser zwischen 0,5 $\mu$m und 5 $\mu$m liegt, Grenzwerte inbegriffen.

10. Verwendung eines Adsorptionsmittels nach einem der Ansprüche 1 bis 9 in den Verfahren des:

   • Trennens von aromatischen C8-Isomerfraktionen und insbesondere der Xylole,
   • Trennens von Isomeren substituierten Tuolens wie Nitrotoluen, Diethyltoluen, Toluendiamin und anderen,
   • Trennens der Cresole,
   • Trennens der mehrwertigen Alkohole.

11. Verwendung nach Anspruch 10 für die Trennung von Paraxylol ausgehend von aromatischen Isomerfraktionen mit 8 Kohlenstoffatomen.

12. Verfahren zur Trennung von Paraxylol ausgehend von aromatischen Isomerfraktionen mit 8 Kohlenstoffatomen, das als Adsorptionsmittel des Paraxylols ein zeolithisches Adsorptionsmittel verwendet, das Barium und/oder Kalium umfasst, das eine äußere Oberfläche, **gekennzeichnet durch** Stickstoffadsorption, größer als 20 $m^2.g^{-1}$, vorzugs-

weise größer als 40 m$^2$.g$^{-1}$ und vorzugsweise noch zwischen 40 m$^2$.g$^{-1}$ und 200 m$^2$.g$^{-1}$ aufweist und vorzugsweiser zwischen 60 m$^2$.g$^{-1}$ und 200 m$^2$.g$^{-1}$ liegt, Grenzwerte inbegriffen, durchgeführt in Verfahren in flüssiger Phase, aber auch in Gasphase, wobei die äußere Oberfläche ausgehend von der Stickstoffadsorptionsisotherme, erhalten durch die t-Plot-Methode bei Anwendung der Norm ISO 15901-3:2007 vor der Adsorption, bestimmt wird, wobei das zeolithische Adsorptionsmittel zwischen 300 °C und 450 °C während einer Dauer, die zwischen 9 Stunden und 16 Stunden liegt, im Vakuum (P < 6,7.10$^{-4}$ Pa) entgast wird,
und der zahlenmäßig mittlere Durchmesser der kristallinen Elemente (oder "Kristalle") zwischen 0,1 $\mu$m und 20 $\mu$m liegt.

**13.** Verfahren nach Anspruch 12, das als Adsorptionsmittel des Paraxylols ein zeolithisches Adsorptionsmittel nach einem der Ansprüche 1 bis 9 verwendet.

**14.** Verfahren nach Anspruch 12 oder Anspruch 13 zum Trennen von Paraxylol ausgehend von Isomerfraktionen aromatischer Kohlenwasserstoffe mit 8 Kohlenstoffatomen in flüssiger Phase durch Adsorption des Paraxylols mit Hilfe eines Adsorptionsmittels nach einem der Ansprüche 1 bis 9 in Anwesenheit eines Desorptionsmittels, das vorzugsweise aus Toluen und *Paradiethylbenzol* ausgewählt ist.

## Claims

**1.** A zeolitic adsorbent comprising at least one FAU zeolite and comprising barium and/or potassium, wherein the external surface area of said zeolitic adsorbent, measured by nitrogen adsorption, is greater than 20 m$^2$.g$^{-1}$, preferably greater than 40 m$^2$.g$^{-1}$, and more preferably comprised between 40 m$^2$.g$^{-1}$ and 200 m$^2$.g$^{-1}$, and even more preferably comprised between 60 m$^2$.g$^{-1}$ and 200 m$^2$.g$^{-1}$ inclusive, the external surface area being determined from the nitrogen adsorption isotherm obtained, by the t-plot method, applying standard ISO 15901-3:2007, and prior to adsorption, the zeolitic adsorbent being degassed between 300°C and 450°C for a time between 9 hours and 16 hours, under vacuum (P < 6.7×10$^{-4}$ Pa), and the number-average diameter of the crystalline elements (or "crystals") being between 0.5 $\mu$m and 20 $\mu$m.

**2.** Zeolitic adsorbent according to claim 1, wherein the content of barium oxide (BaO) is greater than 10%, preferably greater than 15%, very preferably greater than 20%, even more preferably greater than 23%, or even greater than 33 wt% relative to the total weight of the adsorbent, and advantageously, the barium content is between 23% and 42%, and typically between 30% and 40%, inclusive, by weight relative to the total weight of the adsorbent, the content of barium oxide being calculated by elemental analysis of the adsorbent, the elemental analysis being obtained by X-ray fluorescence as described in standard NF EN ISO 12677 : 2011.

**3.** Zeolitic adsorbent according to claim 1 or 2, wherein the content of potassium oxide K$_2$O is below 25%, preferably between 0 and 20%, even more preferably between 0% and 15% and very preferably from 0% to 10% inclusive by weight relative to the total weight of the adsorbent, the content of potassium oxide being calculated by elemental analysis of the adsorbent, the elemental analysis being obtained by X-ray fluorescence as described in standard NF EN ISO 12677 : 2011.

**4.** Adsorbent according to any one of claims 1 to 3, wherein said FAU zeolite has an Si/Al atomic ratio between 1.00 and 1.50 inclusive, preferably between 1.05 and 1.50, preferably between 1.05 and 1.40 inclusive, and even more preferably between 1.10 and 1.40 inclusive, the Si/Al atomic ratio being calculated by elemental analysis of the adsorbent, the elemental analysis being obtained by X-ray fluorescence as described in standard NF EN ISO 12677:2011.

**5.** Adsorbent according to any one of the preceding claims, wherein no zeolitic structure other than a faujasite structure, preferably no zeolitic structure other than the faujasite X structure, is detected by X-ray diffraction.

**6.** Adsorbent according to any one of the preceding claims, wherein the fraction by weight of FAU zeolite, the FAU zeolite preferably being a zeolite X, is greater than or equal to 80% relative to the total weight of adsorbent.

**7.** Adsorbent according to any one of the preceding claims, wherein the total volume of macropores and mesopores, measured by mercury intrusion is comprised between 0.15 cm$^3$.g$^{-1}$ and 0.5 cm$^3$.g$^{-1}$, preferably between 0.20 cm$^3$.g$^{-1}$ and 0.40 cm$^3$.g$^{-1}$, and very preferably between 0.20 cm$^3$.g$^{-1}$ and 0.35 cm$^3$.g$^{-1}$, the total volume of macropores and mesopores being measured by mercury intrusion porosimetry, the sample of adsorbent weighed beforehand, in a cell of the porosimeter, then, after prior degassing at an evacuation pressure of 30 $\mu$m Hg for at least 10 min, filling the cell with mercury at a 0.0036 MPa, and then applying a pressure increasing in stages up to 400 MPa to cause gradual penetration of the mercury into the pore network of the sample, the relation between the pressure applied and the diameter of the pores being established by assuming cylindrical pores, a contact angle between the mercury and the wall of the pores of 140° and a surface tension of mercury of 485 dynes/cm.

8. Adsorbent according to any one of the preceding claims, wherein the volume fraction of macropores is comprised between 0.2 and 1 of the total volume of macropores and mesopores, very preferably between 0.4 and 0.8, and even more preferably between 0.45 and 0.65, inclusive, the total volume of macropores and mesopores being measured by mercury intrusion porosimetry, the sample of adsorbent weighed beforehand, in a cell of the porosimeter, then, after prior degassing at an evacuation pressure of 30 $\mu$m Hg for at least 10 min, filling the cell with mercury at a 0.0036 MPa, and then applying a pressure increasing in stages up to 400 MPa to cause gradual penetration of the mercury into the pore network of the sample, the relation between the pressure applied and the diameter of the pores being established by assuming cylindrical pores, a contact angle between the mercury and the wall of the pores of 140° and a surface tension of mercury of 485 dynes/cm.

9. Adsorbent according to any one of the preceding claims, having a number-average diameter of crystalline elements of between 0.5 $\mu$m and 20 $\mu$m, more preferably between 0.5 $\mu$m and 10 $\mu$m, and even more preferably between 0.5 $\mu$m and 5 $\mu$m, inclusive.

10. Use of an adsorbent according to any one of claim 1 to 9, in the processes selected from :

    - separating cuts of Cs-aromatic isomers and notably of xylenes,
    - separating isomers of substituted toluenes, such as nitrotoluene, diethyltoluene, toluenediamine, and others,
    - separating cresols, and
    - separating polyhydric alcohols.

11. Use according to claim 10, for separating para-xylene from cuts of aromatic isomers with 8 carbon atoms.

12. Method for separating para-xylene from cuts of aromatic isomers with 8 carbon atoms, using; as agent for adsorption of para-xylene, a zeolitic adsorbent comprising barium and/or potassium having an external surface area **characterized by** nitrogen adsorption greater than 20 $m^2 \cdot g^{-1}$, preferably greater than 40 $m^2 \cdot g^{-1}$, and more preferably comprised between 40 $m^2 \cdot g^{-1}$ and 200 $m^2 \cdot g^{-1}$, and even more preferably comprised between 60 $m^2 \cdot g^{-1}$ and 200 $m^2 \cdot g^{-1}$ inclusive, employed in liquid phase processes, but also in gas-phase processes, the external surface area being determined from the isotherm obtained, by the t-plot method, applying standard ISO 15901-3:2007, the external surface area being determined from the isotherm obtained, by the t-plot method, applying standard ISO 15901-3:2007, after degassing at a temperature between 300°C and 450°C for a time comprised between 9 hours and 16 hours, under vacuum (P<6.7$\times$10$^{-4}$Pa), and the number-average diameter of the crystalline elements (or "crystals") is between 0.5 $\mu$m and 20 $\mu$m.

13. Method according to claim 12, using as adsorption agent of para-xylene, a zeolitic adsorbent according to any one of claim 1 to 9.

14. Method according to claim 12 or claim 13, for separating para-xylene from cuts of isomers of aromatic hydrocarbons containing 8 carbon atoms, in the liquid phase, by adsorption of para-xylene by means of an adsorbent according to any one of claims 1 to 9, in the presence of a desorbent, preferably chosen among toluene and para-diethylbenzene.

## RÉFÉRENCES CITÉES DANS LA DESCRIPTION

**Documents brevets cités dans la description**

- US 3558730 A
- US 3558732 A
- US 3626020 A
- US 3663638 A
- US 3960774 A
- US 3878127 A
- US 2985589 A
- FR 2789914
- CN 1267185 C
- US 20090326308 A
- US 7785563 B
- WO 2013106816 A
- WO 2007043731 A
- US 5284992 A
- US 5629467 A
- US 4402832 A
- US 4498991 A
- FR 2789914 A
- FR 2903978

**Littérature non-brevet citée dans la description**

- Principles of Adsorption and Adsorption Processes » (« Principes de l'Adsorption et des Procédés d'Adsorption. John Wiley & Sons, 1984, 326, , 407
- **INAYAT.** *Angew. Chem. Int. Ed.,* 2012, vol. 51, 1962-1965
- **D. VERBOEKEND ; G. VILÉ ; J. PÉREZ-RAMÍREZ.** *Adv. Funct. Mater.,* 2012, vol. 22, 916-928
- **D. W. BRECK.** Zeolite Molecular Sieves. John Wiley and Sons, 1973, 314-315
- **RUTHVEN.** Principles of Adsorption and Adsorption Processes. John Wiley & Sons, 1984